# EUROPEAN PATENT SPECIFICATION

(11) **EP 0 629 131 B1**
(45) Date of publication and mention of the grant of the patent: **27.06.2001**
(21) Application number: 93905659.4
(22) Date of filing: 22.02.1993
(51) Int. Cl.: C12N 15/12, C12N 1/21, C12N 15/70, C12N 15/79, C12N 7/01, C07K 14/435, A61K 39/00, A61K 39/395, C12Q 1/68

(54) **ANTIGENS PROTECTIVE AGAINST (ECHINOCOCCUS GRANULOSUS) INFECTION AND VACCINES CONTAINING SUCH ANTIGENS**
SCHÜTZENDE ANTIGENE GEGEN EINE (ECHINOCOCCUS GRANULOSUS) INFEKTION UND IMPFSTOFFE DIE ENTSPRECHENDE ANTIGENE ENTHALTEN
ANTIGENES PROTECTEURS CONTRE L'INFECTION PAR ECHINOCOCCUS GRANULOSUS ET VACCINS CONTENANT DE TELS ANTIGENES

(30) Priority: 21.02.1992 NZ 24168892
(43) Date of publication of application: 21.12.1994
(73) Proprietor: NEW ZEALAND PASTORAL AGRICULTURE RESEARCH INSTITUTE LIMITED, Hamilton (NZ); THE UNIVERSITY OF MELBOURNE, Parkville, Victoria 3052 (AU)
(72) Inventor: HEATH, David Duncan, Ivey Bay, Paremata (NZ); LAWRENCE, Stephen, Bruce, Upper Hutt (NZ); RALSTON, Mark, John, Upper Hutt (NZ); MAASS, David Richard, York Bay, Wellington (NZ); LIGHTOWLERS, Marshall, William, Williamstown, VIC (AU)
(74) Representative: Armitage, Ian Michael
(86) International application number: NZ9300007
(87) International publication number: WO9316722

(56) References cited:
- WO-A-92/01051
- GB-A- 2 232 675
- MOL. BIOCHEM. PARASITOL., vol. 45, no. 1, 1991 pages 137-146, COUGLE ET AL. 'Molecular cloning of Taenia taeniformis oncosphere antigen genes'
- Molecular and Biochemical Parasitology, Volume 25, No. 2, 1987 (Elsevier/North-Holland Biomedical Press, Amsterdam), SHEPHERD, J.G. et al.: "Specific and Cross, Reactive Antigens of Echinococcus Granulosus Hydatid Cyst Fluid", pages 143 to 154.
- Molecular and Biochemical Parasitology, Volume 37, No. 2, 1989 (Elsevier/North-Holland Biomedical Press, Amsterdam), LIGHTOWLERS, MARSHALL W. et al.: "Subunit Compositions and Specificity of the Major Cyst Fluid Antigens of Echinococcus Granulosus", pages 171 to 182.

## Description

### FIELD OF THE INVENTION

This invention relates to antigens which are protective against, *inter alia, Echinococcus granulosus* infection, to vaccines containing such antigens and to methods of protecting hosts susceptible to *Echinococcus* and *Taenii* parasite infection.

### BACKGROUND OF THE INVENTION

Hydatid disease is caused by infection with the larval (metacestode stage) of tapeworms belonging to the genus *Echinococcus.* Transmission occurs in predator-prey mammalian cycles between carnivorous definitive hosts and herbivorous or omnivorous intermediate hosts.

Of *Echinococcus* tapeworms, *Echinococcus granulosus* is the most significant. *E.granulosus* is the cause of cystic hydatid disease (CHD) and is typified by transmission between the domestic dog and domestic herbivores such as sheep and cattle, with humans also being intermediate hosts, but not normally contributing to the life cycle. As a consequence, *E.granulosus* is geographically widespread and its distribution closely parallels the areas of the world where pastoralism is the main occupation. Therefore, it will be appreciated that CHD is an important endemic disease over large parts of South America, Africa, Australasia, Central Europe, Central Asia and the Mediterranean littoral including North Africa.

Some countries are now attempting control of CHD through education of the dog owner and regular anthelmintic treatment of the dog as definitive host. However, while such programmes have generally been partially successful, eradication has proved more difficult to achieve.

A further desirable approach to control of CHD involves the use of a vaccine against acquisition of the cystic stage of the life cycle of *E. granulosus* by the intermediate host. This approach has the considerable advantage that transmission of the infection to dogs through consumption of cyst-containing offal from the intermediate host is prevented. As a consequence, eradication of the disease becomes a realistic possibility.

It is broadly to the vaccine approach that the present invention is directed.

However, for a commercial vaccine to be developed, it is essential for specific protective antigens against *E. granulosus* to be identified. While investigations to date (Gemmell M A, Immunology 11 325-335 (1966); Heath *et* *al*., J Parasitol 67 (6) 797-799 (1981)) have clearly shown that the oncosphere of *E. granulosus* is a potent source of such antigens, no specific *E. granulosus* oncosphere antigens have been identified as conferring protection.

International Patent Application No. PCT/FR91/00563 (published as WO 92/01051) discloses an immunogenic *E. granulosus* peptide together with the DNA sequence coding for this peptide and methods of both diagnosis and treatment of *E. granulosus* infection using this peptide. However, the disclosed *E. granulosus* antigen (known as "antigen 5") is unrelated to the oncosphere antigens investigated by the applicants. Further, the applicants have shown in their published research that the presence of antibodies to antigen 5 does not protect lambs against challenge infection with *E. granulosus* eggs (Heath *et al.,* Intl Journal of Parasitology 22 1017-1021 (1992)).

### SUMMARY OF THE INVENTION

It is therefore an object of the present invention to provide a specific antigen for use in a vaccine which is protective against *E. granulosus* infection or at least to provide the public with a useful choice.

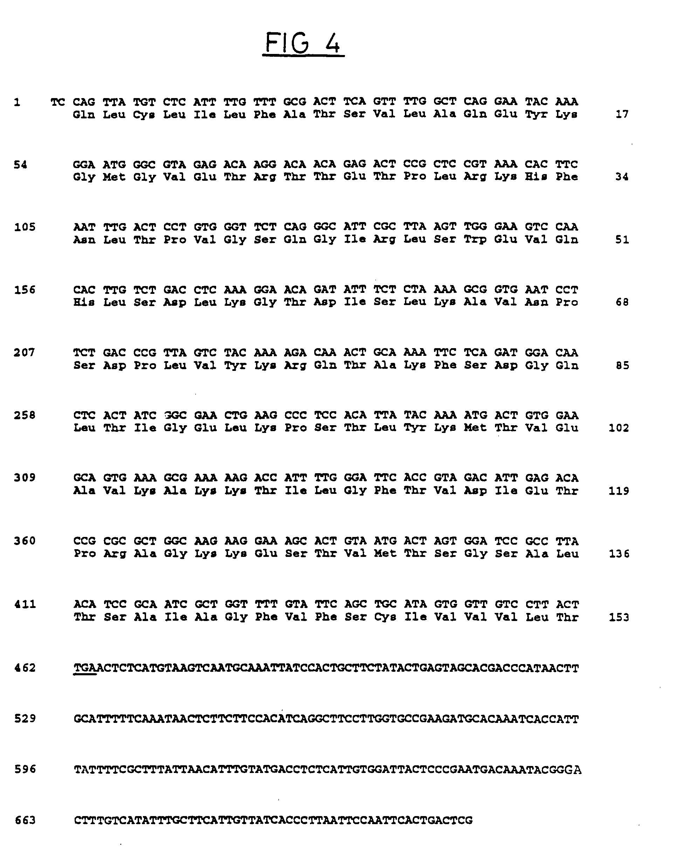

Accordingly, in one aspect the present invention provides, in substantially pure form, an antigenic polypeptide which has a molecular weight in the range of from 23-25 kD calculated by SDS-PAGE, which includes amino acids 4 to 77 from the sequence of Figure 4, and which is capable of generating a protective immunologica response against *E*. *granulosus* infection in a susceptible host; or a peptide fragment or variant having equivalent protective immunological activity thereto.

In a preferred embodiment, the polypeptide includes amino acids 1 to 154 from the sequence of Figure 4.

In still a more preferred embodiment, the invention consists of a peptide fragment having part or all the amino acid sequence of Figure 4.

Conveniently, the polypeptide or peptide fragment is the product of expression of a nucleotide sequence coding therefor in a host cell.

In a further aspect, the invention consists in a composition of matter capable of generating a protective immunological response to *E. granulosus* infection in a susceptible host which essentially consists of a component selected from the group consisting of:
(a) the polypeptide defined above;
(b) a peptide fragment of (a) having equivalent protective immunological activity thereto; and
(c) a variant of (a) or (b) which has been modified by the insertion, substitution or deletion of one or more amino acids and which has at least equivalent immunological activity thereto.

In still a further aspect, the invention provides a DNA molecule which is selected from the group consisting of:
(a) a nucleotide sequence encoding the antigenic polypeptide defined above;
(b) a nucleotide sequence encoding a peptide fragment of the antigenic polypeptide of (a), which fragment has equivalent protective immunological activity to the polypeptide of (a); and
(c) a nucleotide sequence encoding a variant of the polypeptide of (a) or the peptide of (b) in which the amino acid sequence has been modified by the insertion, substitution or deletion of one or more amino acids, which variant has equivalent protective immunological activity to the polypeptide of (a) or the peptide of fragment (b).

In a preferred embodiment, the DNA molecule includes at least nucleotides 10 to 233 of the Figure 4 sequence, more preferably nucleotides 3 to 461 of the sequence of Figure 4, and most preferably nucleotides 1 to 461 of the sequence of Figure 4.

In yet further aspects, the invention provides recombinant expression vectors which contain a DNA molecule as defined above, host cells transformed with such vectors and capable of expressing the polypeptide or peptide fragment or variant thereof which is encoded, and methods of producing an antigenic polypeptide or a peptide fragment or variant thereof comprising culturing a host cell as defined above and recovering the expressed product.

In another aspect, the present invention provides a vaccine against E. *granulosus* infection comprising the *E. granulosus* polypeptide, peptide fragment or variant defined above, together with an immunologically appropriate adjuvant or carrier.

In still another aspect, the present invention provides a method of protecting a susceptible host against *Echinococcus* or *Taeniid* parasite infection comprising the step of administering to said host an amount of polypeptide, peptide fragment or variant defined above which is protective against such infection.

Conveniently, the polypeptide, peptide fragment or variant is administered to said host in the form of a vaccine as defined above.

In a further aspect, the invention provides an antibody specific for the antigenic polypeptide or peptide fragment or variant thereof as defined above.

Other embodiments of the invention will become apparent from the description which follows.

### DESCRIPTION OF THE FIGURES

While the invention is broadly as defined above, it will be appreciated by those persons skilled in the art that the present invention is not limited thereto but that it also includes embodiments of which the following description provides examples. In particular, a better understanding of the present invention will be gained by reference to the accompanying drawings in which:

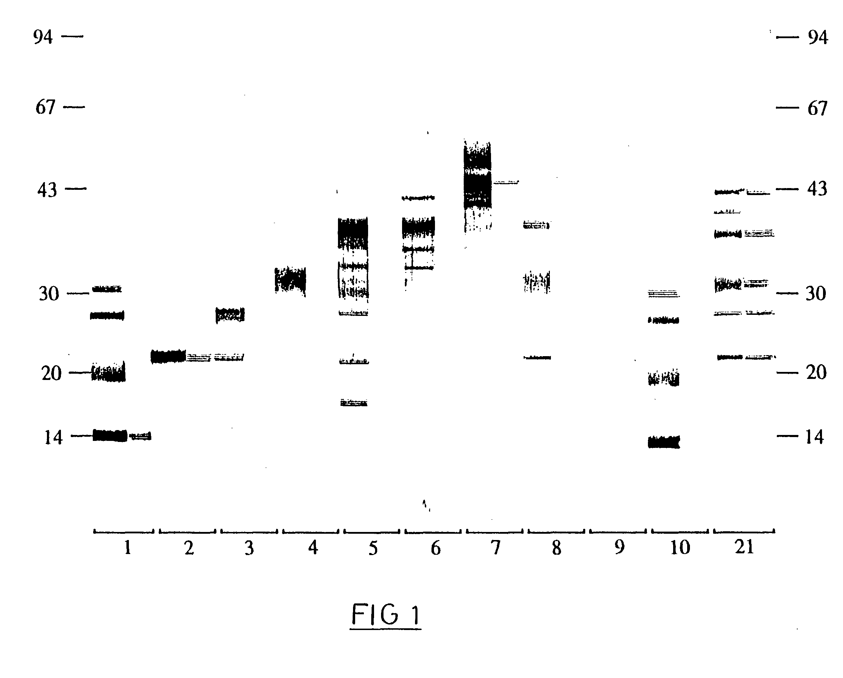

Figure 1 shows IgG₁ and IgG₂ sheep antibody responses to vaccination with preparative SDS-PAGE fractions of oncospheres. Groups of sheep were vaccinated with fractions of oncospheres comprising SDS-PAGE fractions which include antigens of estimated molecular weight: Group 1: All molecules below 21 kD; Group 2: 23 + 25 kD; Group 3: 30 kD; Group 4: 34 kD; Group 5: 40 kD doublet; Group 6: 43 kD; Group 7: All molecules above 43 kD; Group 8: A mix of all molecules; Group 9: Adjuvant only; Group 10 sheep were vaccinated with un-fractionated oncospheres otherwise treated in a similar manner to groups 1 to 9, and Group 21 were vaccinated with a Freeze-Thaw sonicate of activated oncospheres of *E*. *granulosus.* Figure 1 shows analysis of the antibody responses of sheep in each of these groups reacted against whole oncosphere extracts in SDS-PAGE.

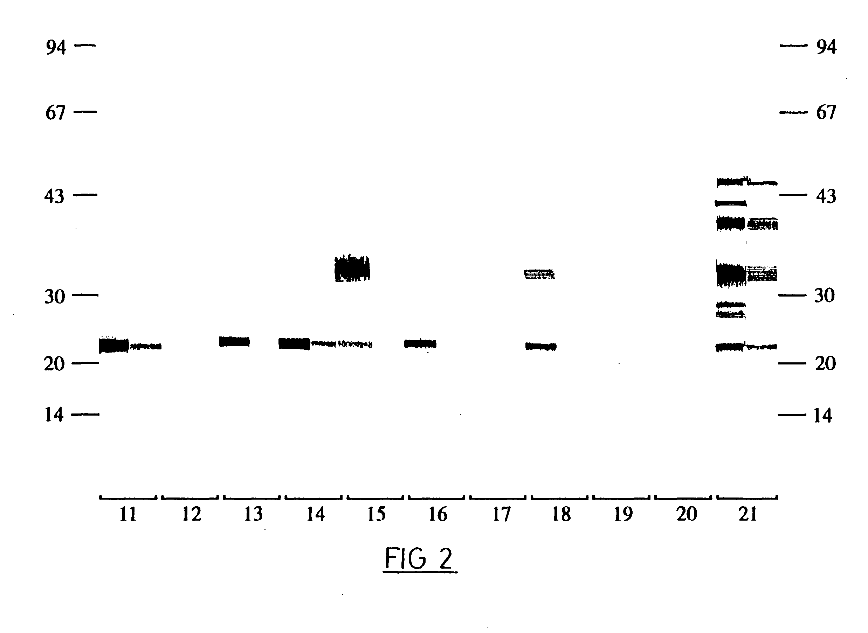

Figure 2 shows IgG₁ and IgG₂ sheep antibody responses to vaccination with cloned antigens. Groups: 11. Clone S2; 12. Clone S3; 13. Clone 48; 14. Clone 95; 15. Clone 101; 16. Clone 119; 17. Clone 123; 18. Mix of all clones; 19. Clones 33, 42 and 85; 20. GST-control; 21. Pooled serum from 5 sheep immunised with a Freeze-Thaw sonicate of activated oncospheres of *E. granulosus.* The immunisation procedure was identical to that following for the antigens of groups 11 to 20.

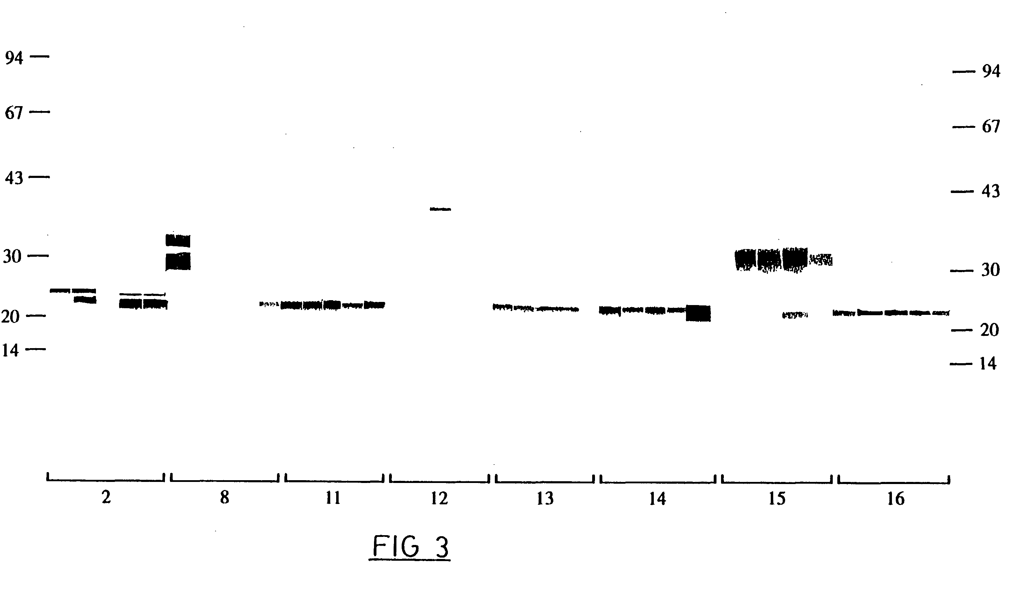

Figure 3 shows sheep IgG₂ responses to vaccination with preparative SDS-PAGE fractions of oncosphere antigens or with cloned antigens. Groups as above.

Figure 4 shows the nucleotide sequence and deduced amino acid sequence of a peptide fragment of the protective antigen of the invention. Numbers to the left hand side indicate nucleotide number from 5' to 3'; numbers to the right hand side indicate amino acid number. The translation stop codon is underlined.

### DETAILED DESCRIPTION OF THE INVENTION

As defined above, in its primary aspect, the present invention is directed to the provision of an antigen which is host-protective against *E. granulosus* infection irrespective of whether this is the worm or cystic stage of such infection. Hosts which are susceptible to *E. granulosus* infection are mammals including humans. Accordingly, examples of hosts to which the invention has potential application are ovine, bovine, porcine, caprine, equine and human hosts.

From their investigations, the applicants have identified an *E. granulosus* polypeptide as being involved in protection against *E. granulosus* infection in a susceptible host. This *E. granulosus* polypeptide is that having a molecular weight in the range of approximately 23-25 kD.

The present invention also includes within its scope antigens derived from the native *E. granulosus* polypeptide identified above where such derivatives have host-protective activity. These derivatives will normally be peptide fragments of the native polypeptide which include the protective epitope, but can also be functionally equivalent variants of the native polypeptide modified by well known techniques such as site-specific mutagenesis (see Adelman *et al*., DNA 2 183 (1983)). For example, it is possible by such techniques to substitute amino acids in a sequence with equivalent amino acids. Groups of amino acids known normally to be equivalent are:

| | | | | | |
|---|---|---|---|---|---|
| (a) | Ala | Ser | Thr | Pro | Gly; |
| (b) | Asn | Asp | Glu | Gln; | |
| (c) | His | Arg | Lys; | | |
| (d) | Met | Leu | Ile | Val; and | |
| (e) | Phe | Tyr | Trp. | | |

In a preferred embodiment, the antigen is a peptide fragment having part or all of the amino acid sequence of Figure 4. More preferably, the peptide fragment will comprise amino acids 4 to 77 from the Figure 4 sequence. Still more preferably, the peptide fragment will comprise amino acids 1 to 77 from the Figure 4 sequence. Most preferably, however, the peptide fragment will comprise amino acids 1 to 153 from the Figure 4 sequence.

The protective antigen of the invention can be produced by isolation from the native *E. granulosus* oncosphere complement using conventional purification techniques. However, it is recognised that for production of the antigen in commercial quantities, production by synthetic routes is desirable. Such routes include the stepwise solid phase approach described by Merryfield (J Amer Chem Soc 85 2149-2156 (1963)) and production using recombinant DNA techniques. The latter route in particular is being employed by the applicants.

In a further aspect, the invention accordingly relates to the recombinant production of the antigenic polypeptide or peptide defined above.

Stated generally, the production of the protective antigen of the invention by recombinant DNA techniques involves the transformation of a suitable host organism or cell with an expression vector including a DNA sequence coding for the antigen, followed by the culturing of the transformed host and subsequent recovery of the expressed antigen. Such techniques are described generally in Sambrook *et al*., "Molecular Cloning", Second Edition, Cold Spring Harbour Press (1987).

An initial step in the method of recombinantly producing the antigen involves the ligation of a DNA sequence encoding the antigen into a suitable expression vector containing a promoter and ribosome binding site operable in the host cell in which the coding sequence will be transformed. The most common examples of such expression vectors are plasmids which are double stranded DNA loops that replicate autonomously in the host cell. However, it will be understood that suitable vectors other than plasmids can be used in performing the invention.

Preferably, the host cell in which the DNA sequence encoding the polypeptide is cloned and expressed is a prokaryote such as *E.coli.* For example, *E.coli* DH5 (Raleigh E A *et al.*, Nucleic Acid Research 16 (4) 1563-1575 (1988)), *E. coli* K12 strain 294 (ATCC 31446), *E. coli B, E. coli* X1776 (ATCC 31537), *E. coli* strain ST9 or *E. coli* JM 101 can be employed. Other prokaryotes can also be used, for example bacilli such as *Bacillus subtilis* and enterobacteriaceae such as *Salmonella typhimurium*, *Serratia marcesans* or the attenuated strain Bacille Calmette-Guerin (BCG) of *Mycobacterium bovis.*

In general, where the host cell is a prokaryote, expression or cloning vectors containing replication and control sequences which are derived from species compatible with the host cell are used. The vector may also carry marking sequences which are capable of providing phenotypic selection in transformed cells. For example, *E. coli* has commonly been transformed using pBR322, a plasmid derived from an *E. coli* species (Bolivar *et al*., Gene 2 95 (1977)). pBR322 contains genes for ampicillin and tetracycline resistance and thus provides easy means for identifying transformed cells.

For use in expression, the plasmid including the DNA to be expressed contains a promoter. Those promoters most commonly used in recombinant DNA construction for use with prokaryotic hosts include the β-lactamase (penicillinase) and lactose promoter systems (Chang *et al*., Nature 275 615 (1978); Itakura *et al*., Science 198 1056 (1977); Goeddel *et al*., Nature 281 544 (1979)) and a tryptophan (trp) promoter system (Goeddel *et al*., Nucleic Acids Res 8 4057 (1980); EPO Publ No. 0036776). While these are the most commonly used, other microbial promoters such as the tac promoter (Amann *et al*., Gene 25 167-178 (1983)) have been constructed and utilised, and details concerning their nucleotide sequences have been published, enabling a skilled worker to ligate them functionally in operable relationship to genes in vectors (Siebenlist *et al*., Cell 20: 269 (1980)).

In addition to prokaryotes, eukaryotic microbes, such as yeast may also be used. *Saccharomyces cerevisiae,* or common baker's yeast is the most commonly used among eukaryotic microorganisms, although a number of other strains are commonly available. For expression in *Saccharomyces,* the plasmid YRp7, for example, (Stinchcomb *et al*., Nature 282, 39 (1979); Kingsman *et al*., Gene 7, 141 (1979); Tschemper *et al*., Gene 10, 157 (1980)) is commonly used. This plasmid already contains the trp1 gene which provides a selection marker for a mutant strain of yeast lacking the ability to grow in tryptophan, for example ATCC No. 44076 or PEP4-1 (Jones, Genetics 85, 12 (1977)). The presence of the trp1 lesion as a characteristic of the yeast host cell genome then provides an effective environment for detecting transformation by growth in the absence of tryptophan.

Suitable promoting sequences in yeast vectors include the promoters for 3-phosphoglycerate kinase (Hitzeman *et al.,* J Biol Chem 255, 2073 (1980)) or other glycolytic enzymes (Hess *et al.,* J Adv Enzyme Reg 7 149 (1968); Holland *et al*., Biochemistry 17 4900 (1978). Other promoters, which have the additional advantage of transcription controlled by growth conditions, are the promoter region for alcohol dehydrogenase 2, isocytochrome C, acid phosphatase, degradative enzymes associated with nitrogen metabolism, and the aforementioned glyceraldehyde-3-phosphate dehydrogenase, and enzymes responsible for maltose and galactose utilisation. Any plasmid vector containing yeast-compatible promoter, origin of replication and termination sequences is suitable.

In addition to microorganisms, cultures of cells derived from multicellular organisms such as mammals and insects may also be used as hosts. In principle, any such cell culture is workable, whether from vertebrate or invertebrate culture. However, interest has been greatest in vertebrate cells, and propagation of vertebrate cells in culture (tissue culture) has become a routine procedure in recent years (Tissue Culture, Academic Press, Kruse and Patterson, editors (1973)). Examples of such useful host cell lines are VERO and HeLa cells and Chinese hamster ovary (CHO) cells. Expression vectors for such cells ordinarily include (if necessary) an origin of replication, a promoter located upstream from the gene to be expressed, along with any necessary ribosome binding sites, RNA splice sites, polyadenylation sites, and transcriptional terminator sequences.

For use in mammalian cells, the control functions on the expression vectors are often provided by viral material. For example, commonly used promoters are derived from polyoma, Adenovirus 2, and most frequently Simian Virus 40(SV40). The early and late promoters of SV40 virus are particularly useful because both are obtained easily from the virus as a fragment which also contains the SV40 viral origin of replication (Fiers *et al*., Nature 273, 113, (1978)). Smaller or larger SV40 fragments may also be used, provided there is included the approximately 250 bp sequence extending from the HindIII site toward the BgII site located in the viral origin of replication. Further, it is also possible, and often desirable, to utilize promoter or control sequences normally associated with the desired gene sequence, provided such control sequences are compatible with the host cell systems.

An origin of replication may be provided either by construction of the vector to include an exogenous origin, such as may be derived from SV40 or other viral (e.g. Polyoma, Adeno, VSV, BPV) source, or may be provided by the host cell chromosomal replication mechanism. If the vector is integrated into the host cell chromosome, the latter is often sufficient.

Upon transformation of the selected host with an appropriate vector, the antigenic polypeptide or peptide encoded can be produced by culturing the host cells. The fusion protein is then recovered.

Following recovery of the antigenic polypeptide or peptide it is purified as desired. The purification procedure adopted will of course depend upon the degree of purity required for the use to which the polypeptide or peptide is to be put. For most vaccination purposes, separation of the fusion protein from most of the remaining components of the cell culture is sufficient as the antigen can be incorporated into a vaccine in a relatively crude form. However, in cases where a greater degree of purity is desired, the carrier component of the fusion protein can be cleaved from the antigenic component. As will again be apparent from the specific examples provided, this can be easily achieved through the provision of an appropriate enzyme cleavage site between the carrier component and the antigen.

Where as is preferred, recombinant techniques are used to produce the antigenic peptide, the first step is to obtain DNA encoding the desired product. Such DNA molecules comprise still a further aspect of this invention.

The DNA molecule of the invention preferably includes at least part of or all of the nucleotide sequence of Figure 4. In one embodiment, the DNA molecule will include at least nucleotides 10 to 233 of the Figure 4 sequence. In a more preferred embodiment, the DNA molecule will include at least nucleotides 3 to 233 of the Figure 4 sequence. It is, however, most preferred that the DNA molecule comprise at least nucleotides 3 to 461 of the Figure 4 sequence if not the entire nucleotide sequence of Figure 4.

The DNA molecule of the invention can be obtained contained within a DNA molecule isolated from an appropriate natural source or can be produced as intron-free cDNA using conventional techniques such as those used in the specific description set out hereinafter. cDNA is preferred.

However, as indicated above, the invention also contemplates variants of the polypeptide which differ from the native amino acid sequences by the insertion, substitution or deletion of one or more amino acids. Where such a variant is desired, the nucleotide sequence of the native DNA molecule is altered appropriately. This alteration can be made through elective synthesis of the DNA using an appropriate synthesizer such as the Applied Biosystems DNA Synthesizer or by modification of the native DNA by, for example, site specific or cassette mutagenesis.

Once obtained, the DNA molecule is treated to be suitable for insertion together with the selected control sequence into the appropriate cloning and/or expression vector. To this end the DNA is cleaved, tailored and religated as required.

Cleavage is performed by treating with restriction enzyme(s) in a suitable buffer. Any of the large number of commercially available restriction enzymes can be used as specified by the manufacturer. After cleavage, the nucleic acid is recovered by, for example, precipitation with ethanol.

Tailoring of the cleaved DNA is performed using conventional techniques. For example, if blunt ends are required, the DNA may be treated with DNA polymerase I (Klenow), phenol and chloroform extracted, and precipitated by ethanol.

Re-ligation can be performed by providing approximately equimolar amounts of the desired components, appropriately tailored for correct matching, and treatment with an appropriate ligase (eg T₄ DNA ligase).

In addition to the protective antigens of the invention and the method of producing these, the present invention provides a vaccine against *E*. *granulosus* infection. Such a vaccine includes as the essential component a host protective amount of the *E. granulosus* polypeptide, peptide fragment or variant referred to above, together with a suitable adjuvant or carrier.

Examples of suitable adjuvants known to those skilled in the art are saponins (or derivative or related material), muramyldipeptide, trehalose dimycollate, Freund's complete adjuvant, Freund's incomplete adjuvant, other water in oil emulsions, double emulsions, dextran, diethylaminoethyl-dextran, potassium alum, aluminium phosphate, aluminium hydroxide, bentonite, zymosan, polyelectrolytes, retinol, calcium phosphate, protamine, sarcosine, glycerol, sorbitol, propylene glycol, fixed oils and synthetic esters of higher fatty acids. Saponins in particular have been found to be effective adjuvants.

In still further embodiments, the vaccine may also be formulated to further include other host-therapeutic agents. Such therapeutic agents include anthelmintics or other vaccines, or immunostimulants such as interferons or interleukins.

The vaccine can be administered to the host by any of those methods known in the art. However, the preferred mode of administration of the vaccine is parenteral. The term "parenteral" is used herein to mean intravenous, intramuscular, intradermal and subcutaneous injection. Most conveniently, the administration is by subcutaneous injection.

The amount of the vaccine administered to the host to be treated will depend on the type, size and body-weight of the host as well as on the immunogenicity of the vaccine. Conveniently, the vaccine is formulated such that relatively small dosages of vaccine (1-5 ml) are sufficient to be protective.

The vaccine may also be in the form of a live recombinant viral vaccine including nucleic acid encoding the polypeptide, peptide fragment or variant. The vaccine is administered to the host in this form and once within the host expresses the encoded polypeptide, peptide fragment or variant to induce a host-protective response.

A number of such live recombinant viral vaccine systems are known. An example of such a system is the *Vaccinia* virus system (US Patent 4603112; Brochier *et al*., Nature 354 520 (1991)).

In still a further aspect, the invention provides a method of protecting a host susceptible to infection by an *Echinococcus* or *Taeniid* parasite. The method of invention includes as its essential step the administration to the host of either the antigenic polypeptide or peptide fragment or variant *per se,* or of a vaccine as described above.

While the specific examples provided hereinafter describe only the use of the antigens of the invention in protecting against *E. granulosus* infection, those persons skilled in the art will appreciate that cross-species protection can be achieved by the use of antigens derived from one parasite species (see, for example, Lightowlers, Acta Leidensia 57 135-142 (1989)). It will therefore be understood that the antigens of the invention are candidate protective antigens against at least the following *Echinococcus* or *Taeniid* parasites other than *E. granulosus: E. multilocularis, E. vogelii, T. ovis, T. saginata, T. solium, T. multiceps* and *T. hydatigena.*

As yet a further aspect of the invention, the use of the DNA molecule described above or a subsequence thereof as a probe is contemplated. In this aspect, the DNA molecule is used to identify by hybridisation DNA of an *Echinococcus* or *Taeniid* parasite such as *T. saginata, T. hydatigena* or *E. multilocularis* which encodes an immunogenic antigen of that parasite. In this way, further parasite antigens suitable for use in a vaccine can be identified.

The method of use of the DNA molecule of the invention as a probe will be well understood by those persons skilled in the art. For example, those techniques set out in Maniatis *et al*., "Molecular Cloning: A Laboratory Manual", Cold Spring Harbour (1982) could be used.

Alternatively, DNA amplification techniques such as the polymerase chain reaction (Saiki *et al*., Science 239 487 (1988)) can be employed to detect homologous DNA of other parasites, with the PCR primers being based upon the nucleotide sequence of Figure 4.

Similarly to the use of the DNA molecules of the invention to identify DNA encoding the corresponding protective antigen of other parasites, antibody probes specific for the protective antigens of the invention can be used to screen the antigens expressed by organisms transformed by the DNA of the parasite in question. The location of a positive clone (one expressing an antigen recognised by the antibody) will allow identification of both the protective antigen itself and the DNA which encodes it. Such antibody probes can be either polyclonal or monoclonal and can be prepared by any of those techniques known in the art. In particular, monoclonal antibodies can be prepared in accordance with the procedure of Kohler and Milstein (Kohler G and Milstein C, "Continuous cultures of fused cells secreting antibody of predifined specificity", Nature 256 495-497 (1975)).

The immunogenicity of the antigenic polypeptide of the invention and of peptide fragments of this polypeptide will be appreciated from the following nonlimiting examples.

### EXAMPLE 1: TESTING OF ANTIGENS PREPARED FROM ECHINOCOCCUS GRANULOSUS ONCOSPHERES BY PREPARATIVE SDS/PAGE

Previous unreported investigations by the applicants resulted in the identification of a number of candidate protective antigens from *E. granulosus*. These candidate antigens had the following molecular weights as determined by SDS-PAGE:
- 23-25 kD
- 30 kD
- 34 kD
- 40 kD

This experiment was conducted by the applicants to identify the host-protective antigen(s) from amongst the above candidates.

### MATERIALS AND METHODS

Preparative Gel: Using a Biorad SDS Preparative Cell, 50 ml of a 15% polyacrylamide solution was poured into a 32 mm holder to a height of 5.5 cm. After setting, 8 ml of stacking gel was poured on top, and allowed to set.

Antigen: Nine million hatched oncospheres were prepared as follows: *E. granulosus* eggs with fully-developed embryophores were counted, and the required number of eggs, assuming a 20% yield of activated oncospheres, were placed in 15 ml disposable plastic centrifuge tubes (Falcon Plastics). No more than 500,000 were placed in each tube. Eggs were centrifuged at 200 g for 2 minutes, the supernatant discarded, and 10 ml of artificial gastric fluid (AIF), at 37 C (Heath D D and Smyth J D, *In vitro* cultivation of *Echinococcus granulosus, Taenia hydatigena, T. ovis, T. pisiformis* and *T. serialis* from oncosphere to cystic larva, Parasitology 61 329-343 (1970)) which had been passed through a 0.2 micron membrane, was added. Tubes were mixed on a rotator at 37 C for 1 h.

Eggs were centrifuged at 200 g for 2 min, the supernatant withdrawn, and replaced by 10 ml of artificial intestinal fluid (AIF) at 37 C (Heath & Smyth, 1970, supra), which had been passed through a 0.2 micron membrane. Eggs were mixed on a rotator at 37 C for 30 min, followed by centrifugation at 1000 g for 2 min. The supernatant was discarded, and replaced by 15 ml of Percoll (Pharmacia, Sweden) diluted aseptically 9:1 (v/v) with 10 x concentrated NCTC135 (GIBCO, NY). The embryophoric blocks, and activated and unactivated oncospheres, which all constituted the pellet, were mixed with the Percoll by inversion. The tubes were then centrifuged at 1000 g for 10 min, and each supernatant, which contained the oncospheres, was tipped into a new sterile 15 ml centrifuge tube. Half of the supernatant (7.5 ml) was then tipped into another sterile centrifuge tube, and to each of the two tubes was added 7.5 ml of NCTC 135. The tubes were mixed by inversion, and centrifuged at 1000 g for 5 min. The supernatants were removed, and the pellet containing oncospheres was washed 2 x with 10 ml of NCTC 135, centrifuging for 2 min at 1000 g each time. The pellet was finally suspended in an appropriate volume of NCTC135 that would give 10 microlitre samples containing between 40 and 200 oncospheres. The number of activated and unactivated oncospheres was then estimated by counting the entire volume of 4 x 10 microlitre samples dispensed onto both sides of two haemocytometer slides (Improved Neubauer), using 200 x magnification to distinguish unactivated oncospheres from those free of their oncospheral membranes (activated oncospheres).

Separation of oncosphere proteins: The oncospheres (50% unactivated and 50% activated approx.) were boiled in SDS sample buffer (Laemmli U K, Cleavage of structural proteins during the assembly of the head of bacteriophage T4, Nature (London) 277 680-685 (1970) (containing Dithiothreitol (DTT), Sigma [10 mg/ml] instead of mercaptoethanol), and loaded onto the Biorad SDS Preparative Cell column. When the bromophenyl blue dye-front had almost reached the bottom of the column, successive 2.5 ml fractions were collected for 24 h.

Enzyme inhibitors: All were obtained from Sigma. They were made up in 20 mM Tris/HCl, pH 8.0, so that when diluted v/v, the final concentrations were: lodoacetamide, 20 mM; Aprotinin, 10 µl/ml; Pepstatin, 2 µg/ml;
N-tosyl-l-phenylalanine chlor methyl ketone (TPCK); 50 *µ*g/ml; Na-p-tosyl-l-lysine-chloro methyl ketone (TLCK), 50 µg/ml; Ethylene diamine tetra-acetic acid (EDTA), 2 mM: Phenyl methyl sulphonyl fluoride (PMSF), 1 mM). They were added to all fractions. A sample from each fraction was run on a 5-25% gel using SDS/PAGE, and the gel was silver-stained to identify molecules of relevant molecular weights. Selected fractions were concentrated in a stirred cell on an Amicon UM3 membrane, to 10 ml. They were then centrifuged at 2200 rpm for 30 m at 1C. The supernatant was removed from the deposit of SDS, and each was then dialysed against 4 changes of 20 mm Tris-buffered saline containing enzyme inhibitors, pH 7.5, over 3 days.

The molecular weights to be tested were selected according to molecular weight fractions as follows: 1. (all molecules below 21 kD); 2. (23 + 25 kD); 3. (30 kD); 4. (34 kD); 5. (40 kD doublet); 6. (43 kD); 7. (all molecules above 43kD); 8. (a mix of all molecules); 9. (adjuvant only); 10. (a sample of the antigen before it was loaded onto the gel. This sample was also treated by cold centrifugation and extensive dialysis).

Immunisation Procedure: Each antigen existed as 10 ml of supernatant. Each fraction represented the particular molecule(s) from 5 million oncospheres, while the mix of all consisted of all the molecules from 2 million, as did the antigen prior to loading (group 10). For the first injection, 1 ml of antigen was homogenised with 1 ml of STM adjuvant (Bokhout *et al*., Veterinary Immunology and Immunopathology 2 491-500 (1981)), and half injected subcutaneously over the left ribs, while the other half was injected intramuscularly into the left rear leg. For the second injection, which was given 1 month later, the formulation and route of injection was the same, except that 2 mg of freeze-dried *Mycobacterium phlei* was included with each injection. Injections were given on the right side.

Challenge with eggs: One month after the second injection, all lambs were given 1000 *E. granulosus* eggs orally. The eggs had been stored at 4°C for 6 weeks. They had been extracted from worms harvested from experimentally-infected dogs, following the method of Heath D D and Lawrence S B, Daily egg-production of dogs infected with *Echinococcus granulosus,* Archivos de la Hidatidosis 30 321-328 (1991).

Experimental animals: Lambs were both Romney and Dorset breeds, randomised through all groups. They had been reared free of infection with *E. granulosus*, and were given their first injection when 8 months old. 10 ml of blood was collected for serum from each animal, using vacutainers, at the time of the first and second injections, and again when the animals were challenged. Serum was removed aseptically, and stored at -20°C until required for testing by *in vitro* culture of oncospheres, or immunoblotting.

Necropsy: Six months after the challenge infection, another blood sample was taken, and then lambs were exsanguinated after stunning with a captive-bolt pistol. The liver and lungs of each animal were removed, and finely sliced to assess the number and condition of cysts present. The liver was sliced at 2-3 mm intervals, and each slice carefully inspected by eye. The lungs were sliced at 4 mm intervals, and cysts were palpated as well as being inspected by eye.

Immunoblotting: *Echinococcus granulosus* oncosphere antigens were separated by SDS PAGE according to published methods (Laemmli (1970) supra; Hames B D and Rickwood D, Gel electrophoresis of Proteins: a practical approach, IRL Press, Oxford (1981)). Samples were solubilised by boiling for 3 min in 60 mM Tris/HCl pH 6.8, 10% glycerol, 2% SDS, 1 % DTT and 0.05% bromophenol blue. The separation took place on a gradient gel (T = 5-25%) using a vertical electrophoresis system (BioRad Protean 11), following the manufacturer's instructions. Separated antigens were transferred electrophoretically (Towbin H, Staehelin T and Gordon J, Electrophoretic transfer of proteins from polyacrylamide gels to nitrocellulose sheets: Procedure and some applications, Proceedings of the National Academy of Sciences USA 76 4350-4354 (1979)) to nitrocellulose paper (0.45 *µ*m, Schleicher & Schull, Dassell, Germany) in a cooled Transblot cell with plate electrodes (BioRad, Richmond, USA) at 50 v for 2 h in 10 mM carbonate buffer pH 9.0 containing 20% methanol (Dunn S D, Effects of the modification of transfer buffer composition and the renaturation of proteins in gels on the recognition of proteins on Western blots by monoclonal antibodies, Analytical Biochemistry 157 144-153 (1986)). After transfer, nitrocellulose sheets were placed in 0.5% Ponceau S (BDH) in 1% acetic acid for 5 min, then rinsed with distilled water until protein bands were clearly visible. Molecular weight markers (Pharmacia) were marked with pencil. Individual strips of nitrocellulose containing antigen were cut and placed in incubation trays (Schleicher & Schull, Germany). Strips were blocked with 5% fat-free milk powder and 0.1% Tween 20 (BDH) dissolved in 20 mM Tris/HCl pH 7.5, 0.5% NaCl, for 1 h at 37 C. Test sera were diluted 1:100 in the blocking buffer. Strips were incubated in the diluted sera overnight at room temperature on a rocking platform. Strips were then washed with 4 x 10 min changes of 20 mM Tris/HCl pH 7.5 containing 0.5 M NaCl and 0.1% Tween 20.

An extra step was included in the immunoassay to delineate the immunoglobulin classes, IgG₁ and IgG₂. After the first antibody, the strips were probed with mouse monoclonal antibodies to sheep IgG₁ or IgG₂, obtained from Dr Ken Beh, CSIRO McMaster Laboratory, Sydney. The strips were then probed with Goat anti-mouse IgG (whole molecule) labelled with peroxidase [Cappel], diluted 1:1000 in the blocking buffer. The strips were then washed 3 x with the blocking buffer without milk powder or Tween 20. Peroxidase activity was visualised with 3-amino-9-ethylcarbazole (Sigma). Development was stopped on all strips at the same time, so that comparisons could be made of the intensity of the stains, thus reflecting the amount of the specific immunoglobulin in each sera.

Killing of oncospheres *in vitro:* The pooled prechallenge sera from each of the 10 groups of sheep was tested for oncosphere-killing capacity.

For foetal lamb serum complement, foetal lambs close to term were bled with a sterile syringe, and blood was immediately transferred to 50 ml sterile polypropylene tubes (Falcon Plastics). After clotting for 2 h at ambient temperature, clots were separated from the walls with sterile applicator sticks in a laminar-flow sterile cabinet. Tubes were then centrifuged in a Beckman refrigerated centrifuge at 4°C for 30 min. Tubes were stored in ice while serum was removed. Foetal lamb serum was stored in 2 ml aliquots at -70°C until required for culture.

Cultures were established in 96 well flat-bottomed culture plates (Falcon Microtest III). To each well was added 150 µl of test serum (inactivated at 56 C for 30 min), plus 150 *µ*l of NCTC135 containing 50 activated oncospheres, plus 20 µl of foetal lamb serum complement. All cultures were established in duplicate or triplicate. Plates were kept in a humidified CO₂ incubator at 37°C, and results were assessed at 24 h and at 7 days, using a Leitz Labovert inverted microscope. Wherever NCTC135 is mentioned, it was supplemented with 300 mg/l of cysteine hydrochloride (Sigma) and 50 *µ*g/ml of gentamicin sulphate (Schering).

### RESULTS

The antibody profiles against oncosphere antigen that were generated by each group are shown in Figure 1. It is clear that the various groups produced antibodies that predominantly recognised the molecular weight range with which they were immunised. In some groups, such as groups 1 and 5, there were epitopes on the immunising molecules that were shared with molecules of other molecular weights.

The results of necropsy are shown in Table 1, groups 1-10, and indicate that, while the other candidate antigens do reduce the number of cysts relative to the control, it is only the 23-25 kD molecular weight range which induces a significant degree of protection (P = 0.012, F = 10.62, 8DF).

The results of the *in vitro* killing of oncospheres by the sera of groups of sheep immunised with the various molecular weight fractions are shown in Table 2. As can be clearly seen, these results corroborate that it is only the 23-25 kD molecular weight antigen from *E. granulosus* which is host-protective.

**Table 1:**

| Necropsy results of sheep challenged with 1000 *E. granulosus* eggs after immunisation with native antigens eluted from polyacrylamide gels (groups 1-8), adjuvant controls (group 9) or SDS treated oncospheres (group 10) | | | | |
|---|---|---|---|---|
| Group | Immunising Antigen | Sheep Tag Numbers | Number of Cysts | Average |
| 1 | <20 | 62,47,12,50,65 | 75,39,19,101,47 | 56.2 |
| 2 | 23 + 25 | 05,24,67,45,95 | 0,39,10,2,4 | 11 |
| 3 | 30 | 17,19,13,57,03 | 0,42,17,148,197 | 80.8 |
| 4 | 34 | 11,37,14,77,43 | 167,9,48,85,126 | 87 |
| 5 | 40 | 51,39,06,41,82 | 109,86,156,276,97 | 144.8 |
| 6 | 43 | 08,64,04,88,68 | 57,67,165,3,52 | 68.8 |
| 7 | >43 | 86,45,40,72,01 | 84,75,2,17,78 | 51.2 |
| 8 | MIX | 15,23,92,48,27 | 0,0,104,133,79 | 632 |
| 9 | CONTROL | 96,09,38,90,33 | 27,137,170,242,86 | 132.4 |
| 10 | SDS ONCS | 60,30,44,20,18 | 247,49,175,25,54 | 110 |

**Table 2:**

| The *in vitro* killing of *E. granulosus* oncospheres by pooled group sera collected on the day of challenge with *E. granulosus* eggs. | | |
|---|---|---|
| Group Number | Treatment | % Killed |
| 1 | <20 kD | 0% |
| 2 | 23-25 kD | 91% |
| 3 | 30 kD | 0% |
| 4 | 34 kD | 0% |
| 5 | 40 kD | 0% |
| 6 | 43 kD | 0% |
| 7 | >43 kD | 0% |
| 8 | Mix of all molecules | 0% |
| 9 | Adjuvant only | 0% |
| 10 | SDS-oncospheres | 0% |

### EXAMPLE 2: AFFINITY-PURIFICATION OF ANTIBODY PROBES

Affinity-purified antibody from the immobilised 23-25 kD molecule was prepared as follows: Lambs 1488 and 1489 were each injected subcutaneously and intramuscularly with 100,000 activated oncospheres of *E. granulosus* on four occasions. The first two injections were 2 weeks apart, then 1 month later and then 3 months later. One week after the last injection, the lambs were bled, 300 ml of blood yielding 150 ml of sterile "hyperimmune" antiserum each. Lambs were challenged with 3000 freshly collected *E. granulosus* eggs on the day that serum was collected as were two control lambs. At necropsy 6 months later, immunised lambs only had cysts growing at the site of the first injections, whereas controls had large numbers of cysts in their livers and lungs.

Serum from lamb 1488 was used for affinity-purification of antibodies, because it had a higher titre of oncosphere-killing capacity than 1489. Antibodies specific to *E. granulosus* oncosphere Freeze-Thaw-Sonicate antigen, oncosphere SDS/PAGE whole-lane, or to antigens with relative mobilities of 0-20, 20-25, 25-30, 30-36, 36-67 kD, or to antigens of relative mobilities of 0-20, 20-30, 30-43, 43-67, 67-90, 90-180 and > 180 kD, were affinity-purified from nitrocellulose strips containing the above antigens, using the method of Beall J A and Mitchell G F, Identification of a particular antigen from a parasite cDNA library using antibodies affinity purified from selected portions of Western blots, Journal of Immunological Methods 86 217-223 (1986). The carrier protein was 1% foetal lamb serum. Affinity-purified antibodies were washed, and then concentrated 10 x using an Amicon stirred cell and PM10 membrane.

### EXAMPLE 3: RECOMBINANT DNA CLONING AND EXPRESSION OF ECHINOCOCCUS GRANULOSUS ONCOSPHERE ANTIGENS

### 1. Isolation and verification of mRNA

### (a) Preparation of reagents

All solutions used in the isolation and handling of mRNA, except solutions containing Tris [Tris (hydroxymethyl)aminomethane Sigma, St Louis, MO USA], were treated with 0.1% diethylpyrocarbonate (DEPC, Sigma) overnight and subsequently autoclaved prior to use. All glassware was baked at a temperature of 200°C or greater overnight. Tris stock solutions were prepared with DEPC-treated water and autoclaved.

### (b) Oncosphere

Mature *E. granulosus* eggs were obtained and hatch/activated as detailed in Example 1. Approximately 4 x 10⁶ oncospheres were solubilised on ice in nuclease-free 6M guanidine hydrochloride (BRL Ultrapure, Bethesda Research Laboratories, USA), 0.1 M sodium acetate, pH 5.2, using a glass/teflon tissue homogeniser. Solubilised oncospheres were stored at -70°C prior to isolation of RNA.

### (c) Isolation of RNA

The solubilised oncospheres were thawed on ice and insoluble material was pelleted by centrifugation at 10,000 rpm, 30 minutes, 4°C in a Type 50 Ti rotor and Beckman L8 ultracentrifuge (Beckman, Palo Alto CA USA). The supernatant was layered over a solution containing 4.8 M caesium chloride (CsCl - BRL Ultrapure, Bethesda Research Laboratories, Gaithersburg, MD USA), 10 mM EDTA (Ethylenediaminetetra-acetic acid, disodium), 6.5 ml guanidine/5.5 ml CsCl per Beckman SW40 polyallomer tube. Tubes were centrifuged at 35,000 rpm, 18 hr, 15°C in a SW40 rotor, Beckman L8 ultracentrifuge. The supernatant was discarded and the base of the tube rinsed briefly and gently with 200 µl of T₁₀E₁ (10 mM Tris, 1 mM EDTA, pH 8.0). Pelleted RNA was dissolved in 200 µl T₁₀E₁ and precipitated by the addition of 1:10 volume 3M sodium acetate and 2.5 volumes 100% ice cold ethanol and incubated at 20°C overnight. Precipitated RNA was pelleted by centrifugation in a microcentrifuge for 15 min at 4°C, the pellet rinsed with 70% ethanol, centrifugation repeated and the subsequent pellet air dried. RNA was resuspended in 50 µl T₁₀E₁ and the quantity assessed by absorbence at a wavelength of 260 nm to be 23 µg total RNA. One microgram was examined in a 1.2% formaldehyde agarose gel (Sambrook J, Fritsch E F and Maniatis T, Molecular cloning. A laboratory manual. Cold Spring Harbour Laboratory Press (1989)) and the RNA found to include intact ribosomal RNA.

### (d) Purification of poly A⁺ RNA

Poly A+ messenger RNA was purified on oligo (dT) cellulose Type 7 (Pharmacia LKB Biotechnology, Uppsala, Sweden) according to the manufacturer's instructions, especially as described by Aviv H and Leder P, Purification of biologically active globin messenger RNA was by chromatography on oligothymidylic acid-cellulose, Proc Nat Acad Sci 69 1408-1412 (1972). Eluted poly A⁺ mRNA was precipitated as above and the pellet redissolved in 50 µl T₁₀E₁.

### (e) In vitro translation

The mRNA in a 5 *µ*l aliquot was *in vitro* translated using Rabbit Reticulocyte Lysate (Amersham, UK) in a total reaction volume of 25 µl according to the manufacturer's instructions. The products were radiolabelled using 37.5 µCi ³⁵S-methionine (Amersham International, Amersham UK) and examined by polyacrylamide gel electrophoresis in a 10% acrylamide gel under reducing condition (Laemmli U K, Cleavage of structural proteins during the assembly of the head of bacteriophage T4, Nature 227 680-685 (1970)) and detected using fluorography following impregnation of the gel with Amplify (Amersham, UK). Translation products were abundant and of heterogeneous size, including products of more than 94 kD.

### 2. Synthesis and cloning of cDNA

### (a) Synthesis of cDNA

Thirty-seven microlitres of the mRNA solution in T₁₀E₁ was reverse transcribed in the presence of α ³²P-dATP (Amersham) using Moloney-Murine Leukemia Virus Reverse Transcriptase using the reagents and protocol contained in the ZAP-cDNA Synthesis Kit (Lot #UC106, Catalog #200400, Stratagene, La Jolla, USA). Following second strand synthesis, an aliquot was assessed in 1% alkaline agarose gel electrophoresis according to the ZAP-cDNA Synthesis Kit instructions. The cDNA was heterogeneous in size and, in comparison with Lambda *Hind* III DNA markers (Promega Corporation, Madison, WI USA), included detectable transcripts greater than 2000bp.

### (b) Cloning of cDNA

cDNA was cloned into the bacteriophage vector Uni-ZAP XR (λ ZAP) (Stratagene) according to the conditions detailed in the UNI-ZAP XR Cloning Kit manufacturer's instructions (Lot #18D, Catalog #237211, Stratagene). The cDNA was ligated to the vector without any selection as to the size of the cDNA. The entire cDNA sample was ligated to 1 µg vector DNA.

### (c) In vitro packaging of bacteriophage

Two microlitres of the vector ligated cDNA were packaged into infective phage using a single Gigapack II Gold packaging reaction (Lot #1535, Catalog #200216, Stratagene) according to the manufacturer's instructions. The resultant phage was titred on *E. coli* PLK-F'. The remaining ligated cDNA was subsequently packaged in two equal reactions and the products from the three reactions pooled and titred. The resultant library of cDNA clones (cDNA library) in λZAP was titred in *E*. *coli* PLK-F' and assessed to comprise approximately 100,000 primary clones (methods as described in the UNI-ZAP XR Cloning Kit, Stratagene).

### 3. Amplification of the cDNA library

The entire cDNA library was amplified on *E*. *coli* PLK-F' cells at a density of 5,000 plaque forming units (pfu) per 150 mm diameter agar plate. Amplified phage was harvested overnight into 10 ml SM (100 mM NaCl, 8 mM Mg SO₄, 50 mM Tris pH 7.5, 0.01% w/v gelatin). The amplified library was pooled and titred on *E. coli* XL1-Blue cells (Stratagene) in the presence of isopropylthiogalactoside (IPTG) and 5-bromo-4-chloro-3-indolyl-β-D-galactoside (Xgal), according to the instructions in the UNI-ZAP XR Cloning Kit (Stratagene). The amplified library titre was assessed to be 1.7 x 10⁹ pfu per ml.

### 4. Immunoscreening of cDNA library

### (a) Preparation of antibody probes

Two types of antibody probes were used in plaque immunoassay; sera and affinity purified antibodies prepared in accordance with Example 2. Serum samples were depleted of "background" reactivity to either *E*. *coli* and/or β-galactosidase as follows. Non-recombinant λ gt11 or λ ZAP were plated at near-confluence on suitable host *E. coli* (generally XL-1 Blue, Stratagene). Nitrocellulose filters (Schleicher & Schuell, Dassel, Germany) were impregnated with 10 mM IPTG, dried and, following incubation of the plates at 42°C for 4 hours, one filter was overlayed onto each plate surface. Plates were incubated subsequently at 37°C overnight, followed by incubation at 4°C for 30 min. The filters were removed and rinsed in TNT (150 mM NaCl, 0.05% Tween 20, 10 mM Tris, pH 8.0) and incubated in 5% skim milk powder/TNT (BLOTTO) for 1 hour. All immunoassay incubations were at room temperature (RT°) on a rocking platform. Each serum sample to be used in immunoassay was diluted in TNT plus 20% foetal calf serum (FTNT) and incubated in a petri dish containing 20 ml diluted sera plus two nitrocellulose filters prepared as above. Following incubation for 2 hr the nitrocellulose filters were removed and the affinity depleted sera were ready for use in plaque immunoassay.

Antibody probes which had been derived from sera by affinity purification (detailed in Example 2) were not affinity depleted with non-recombinant λ gtll or λ ZAP and were used in immunoassay without dilution.

### (b) Immunoassay of cDNA library clones

Two sources of antibody were used in primary screening of the amplified cDNA library. Sera from Sheep 1488, obtained as described in Example 2, was prepared as above and used to identify cDNA clones expressing *E. granulosus* oncosphere antigens. The cDNA library was plated and immunoassayed using standard procedures as described in Sambrook *et al*. (supra). In brief: Plaques were plated on 150 mm petri dishes at 5000 pfu per LG agarose plate and NZY top agar (0.9% agar). *E. coli* strains were BB4 or XL1 Blue (Stratagene). Clones binding antibody in the test sheep sera were detected using rabbit anti-sheep IgG (heavy and light chain) antibody conjugated with alkaline phosphatase (catalogue No. 313 055 003, Jackson ImmunoResearch Laboratories, West Grove, USA). The antibody conjugate was diluted 1:2000 in BLOTTO and incubated at 5 ml per filter, each filter separately for 2 hr. Filters were subsequently washed separately, with six changes of TNT over a period of 1 hr, with rocking between changes. Positive plaques were detected with the chromogenic substrate bromochloroindolyl phosphate/nitro blue tetrazolium (BCIP/NBT) as described by Harlow E and Lane D, Antibodies. A laboratory manual, Cold Spring Harbour Laboratory Press (1988). A total of 129 clone plaques were identified as showing putatively positive signals with the serum. Each associated plaque was picked from the agar plate using a sterile pasteur pipette and placed into 0.5 ml SM. The SM supernatant from each clone containing the passively eluted clone bacteriophage were titred using standard techniques (Sambrook *et al.,* supra) and re-screened on 75 mm plates and filters at a low density (estimated 50 plaques per plate). Of the 129 original clones, 80 clones were confirmed positive when reacted as above with Sheep 1488 serum.

The library was screened in a similar manner also with antibodies affinity purified from sheep 1488 as also described in Example 2. A single positive clone was detected with serum fraction 2 (corresponds to antibodies to 20-25 kD oncosphere antigens), designated clone S2, and one clone was picked as reacting with serum Fraction 3 (25-30 kD antigens), designated S3. Both clones were confirmed positive in secondary plaque immunoassays as above.

Each clone phage was plated on a single 150 mm plate at 20,000 pfu/plate, and phage stocks collected as described for library amplification above.

### (c) Selection of clones

From the clone library, on the basis of the immunoassay results, clones s2, s3, 48, 95, 101, 119 and 123 were selected for further analysis as to whether the antigens they express were host-protective. Clones 33, 42 and 86 (which previous unreported investigations by the applicants had shown were not able to induce statistically significant protection) were also selected to be used as controls.

### 5. Sub-cloning and expression of cDNA

### (a) Production of pBluescript

The plasmid pBluescript was generated by phagemid rescue from λ ZAP stocks selected for each cDNA clone as described by in the UNI-ZAP XR Cloning Kit manufacturer's instructions (Stratagene).

### (b) Sub-cloning into pGEX-3EX

The vector system chosen for expression of the *E*. *granulosus* antigens for vaccine trials was the pGEX series of plasmids (AMRAD Corporation, Melbourne, Australia) which express the parasite-encoded portion of the molecule as a fusion protein at the carboxyl terminus of the *Schistosoma japonicum* glutathione S-transferase (Smith D B and Johnson K S, Single-step purification of polypeptides expressed in *Escherichia coli* as fusion proteins with glutathione S-transferase, Gene 67 31-40, (1988)). In order to be able to sub-clone the directionally cloned cDNA's from the λ ZAP XR vector, it was necessary to modify the pGEX 3X vector in order to insert a *Xho* 1 restriction site downstream from the *Eco* R1 restriction site in the pGEX polylinker. This was achieved by insertion of annealed oligonucleotides having the following sequence:

5' AAT TCA TAC TCG AGT 3'
3' GT ATG AGC TCA TTA A 5'

In addition to incorporation of the *Xho 1* site, this construct preserved the unidirectional cloning of cDNA's as generated in the λ ZAP XR vector. The modified vector was designated pGEX 3EX. Routine methods were used in this modification of the pGEX vector as detailed in Sambrook *et al.* (supra); briefly, annealed oligonucleotides were treated with T4 Polynucleotide kinase (New England Biolabs, Beverly, CA, USA); pGEX 3X DNA was digested with *Eco* Rl (New England Biolabs), phosphatased and ligated to the kinased, annealed oligonucleotides. *E. coli* JM101 were transformed with the ligated plasmid using the calcium chloride method. Bacterial clones transformed with the plasmid were selected, plasmid DNA isolated and the modification confirmed by sequencing the relevant section of DNA using the dideoxy chain-termination method after sub-cloning of a *Bam* H1 (position 914)/*Pst* 1 (position 1885) into bacteriophage M13. The pGEX 3EX vector has the following DNA sequence and predicted translated amino acid sequence across the polylinker region:

BamH1
pGEX-3EX CCA AAA TCG GAT CTG ATC GAA GGT CGT GGG ATC
Pro Lys Ser Asp Leu Ile Glu Gly Arg Gly Ile
Factor X
Sma1
EcoR1
CCC GGG AAT TCA TAC TCG AGT AAT TCA TCG TGA
Pro Gly Asn Ser Tyr Ser Ser Asn Ser Ser ***

Plasmid pBluescript DNA was purified by centrifugation in CsCl (Sambrook *et al*., supra) and insert cDNA excised as follows. Approximately 2 *µ*g of each pBluescript DNA clone was digested with 10 units each of *Eco*R1 and *Xho*1 (Amersham, UK) in 20 µl high salt buffer. Insert DNA was purified in 1% agarose, the DNA bands excised and purified using Geneclean (BIO101 Inc., La Jolla, USA). Following quantitation by absorbance at 260 nm, approximately 100 ng of insert DNA was ligated (2 units T4 DNA ligase, Boehringer Mannheim) to approximately 50 ng of CsCl purified, phosphatased pGEX-3EX DNA digest with both *Eco*R1 and *Xho*1.

The ligated cDNA in pGEX-3EX (see below) was transformed into *E*. *coli* strain BB4 (Stratagene Cloning Systems, La Jolla, California, USA, catalogue # 200269) by electroporation using a BioRad Gene Pulser according to the manufacturer's instructions (BioRad, Richmond, USA).

### EXAMPLE 4: PRODUCTION OF E. GRANULOSUS FUSION PROTEIN

Selected *E. granulosus* oncosphere cDNA clones were subcloned into the expression plasmid pGEX-3EX and transformed into *E. coli* strain BB4 as described above.

Expression yields a parasite protein fused with glutathione S-transferase (GST). Such fusion proteins are frequently soluble and able to be purified from lysed cells under non-denaturing conditions by absorption with glutathione-agarose beads.

### METHOD AND MATERIALS

GST fusion proteins were induced and purified essentially as described by Smith and Johnson, Gene 67 31-40 (1988) using the specific steps detailed below:
1. A colony of pGEX transformant was inoculated into 4 x 100 ml LB/ampicillin medium, incubated overnight at 37°C in a shaking incubator.
2. Cultures were diluted 1:10 into fresh LB/ampicillin medium, incubated 90 mins at 30°C.
3. Expression was induced by the addition of 100 mM IPTG to 0.1 mM.
4. Incubation was continued an additional 4½ hours.
5. Cells were harvested by centrifugation at 5000 x g, 4°C, 10 mins.
6. The cell pellet from each 1 litre was resuspended in 15 ml cold PBS pH 7.2 and stored at -20°C/-70°C until required.
7. Pellets were thawed and lysozyme added to 0.25 mg/ml, incubated room temperature, 10 mins.
8. Triton X-100 was added to 0.5%.
9. Cell suspensions were sonicated on ice 3 x 12 sec burst, with 15 sec recovery periods.
10. Insoluble material was removed by centrifugation at 10,000 x g, 4°C, 15 mins.
11. The supernatant from 1 litre starting culture was added to 15 ml 50% glutathione agarose beads and mixed gently at room temperature, 3 hours.
12. Agarose beads were pelleted by centrifugation at 2000 g, 5 min, at room temperature.
13. Agarose beads were washed 5 x with the bed volume of cold PBS by centrifugation at 2000 g, 5 min, room temperature.
14. GST fusion protein attached to agarose recovered.
15. Fusion protein concentration on the agarose beads was determined by the Bradford protein assay (Biorad).

### EXAMPLE 5: DEMONSTRATION OF IMMUNOGENICITY OF E. GRANULOSUS FUSION PROTEIN

### IMMUNISATION OF SHEEP

The sheep were 8 months old at the time of the initial vaccination, and had been reared on pasture free of *E. granulosus* eggs. They were a mix of Romney and Dorset breeds, and were randomised into 9 groups of 5 treated sheep and 1 group of 8 controls. Each animal received 50 *µ*g fusion protein attached to agarose (prepared in accordance with Example 4), made up 1 saline:1 STM adjuvant (Bokhout B A, van Gaalen C and Van der Heijden Ph J, A selected water-in-oil emulsion: Composition and usefulness as an immunological adjuvant, Veterinary Immunology and Immunopathology 2 491-500 (1981)). The total injection volume, of 2 ml/sheep was administered half subcutaneously and half intramuscularly on the left side, and was repeated 1 month later on the right side. Controls received the expressed glutathione S-transferase from non-recombinant pGEX.

Two weeks after the second injection, the sheep were bled and serum prepared for *in vitro* culture and for immunoblotting against oncosphere antigen as described in Example 1. The same day, the sheep were challenged with 1000 E. *granulosus* eggs orally, as described for Example 1.

### NECROPSY

Six months after challenge, sheep were exsanguinated after stunning with a captive bolt, arid their liver and lungs removed for examination. Livers were sliced at 2 mm intervals and inspected closely by eye for the presence of cysts. Lungs were sliced at 4 mm intervals and the slices palpated to determine the presence of cysts. All questionable lesions were sliced open to confirm whether they were *E. granulosus* cysts.

### RESULTS

Necropsy results are shown in Table 3, and oncosphere-killing *in vitro* in Table 4. A significant degree of immunity to challenge was stimulated by clone S2 (97%. P< 0.001. F=24.04, 11DF); clone 48 (83%. P=0.002. F=16.51, 11DF); clone 95 (96%. P<0.001. F=23.41, 11DF); clone 119 (83%. P=0.002, 11DF).

*In vitro* killing of oncospheres was only observed in pooled sera from these four clones, arid the mix of all clones.

All these clones produced antibody that reacted with the 23-25 kD native protein, while two clones (101 and 123) that did not induce statistically significant protection reacted with the native 34 kD molecule. Another clone that did not induce statistically significant protection (S3), reacted with the 30 kD molecule (Figure 2).

Clones S2 and 95 both induced a strong IgG₂ response to the 23-25 kD molecule, compared to the less-effective clones 48 and 119. Individual sheep in these groups of five also reinforced the hypothesis that a strong IgG₂ response is helpful in inducing protective immunity (Figure 3).

The small variability in the results of the 10 sheep immunised with clone S2 or 95 indicates that this sequence may be recognised by a wide range of genotypes.

**Table 3:**

| Necropsy results of sheep challenged with *E*. *granulosus* eggs after immunisation with recombinant antigens | | | | |
|---|---|---|---|---|
| Group | Immunising Antigen | Sheep Tag Numbers | Number of Cysts | Average |
| 11 | CLONE S2 | 28,53,46,29,16 | 2,3,0,16,3 | 4.8 |
| 12 | CLONE S3 | 56,54,36,26,71 | 0,68,72,53,99 | 58.4 |
| 13 | CLONE 48 | 31,49,25,22,66 | 17,58,12,0,46 | 26.6 |
| 14 | CLONE 95 | 76,80,10,42,52 | 13,14,5,0,2 | 6.8 |
| 15 | CLONE 101 | 58,02,93,79,83 | 64,79,30,108,100 | 76.2 |
| 16 | CLONE 119 | 84,73,91,85,55 | 53,29,0,13,40 | 27 |
| 17 | CLONE 123 | 21,07,34,81,63 | 305,8,100,20,40 | 94.6 |
| 18 | MIX of 7 CLONES | 89,61,75,74,70 | 8,74,27,0,105 | 42.8 |
| 19 | CLONES 33,42,86 | 32,87,69,78,59 | 6,130,136,5,200 | 95.4 |
| 20 | GST-CONTROL | 94,97,99,00,01,02,04, 05 | 70,105,270,180, 122,227,108,171 | 156.6 |

**Table 4:**

| The *in vitro* killing of *E. granulosus* oncospheres by pooled group sera collected on the day of challenge with *E. granulosus* eggs. | | |
|---|---|---|
| Group Number | Treatment | % Killed |
| 11 | Clone S2 | 94% |
| 12 | Clone S3 | 0% |
| 13 | Clone 48 | 97% |
| 14 | Clone 95 | 100% |
| 15 | Clone 101 | 0% |
| 16 | Clone 119 | 89% |
| 17 | Clone 123 | 0% |
| 18 | Mix of all clones | 89% |
| 19 | Mix of clones 33,42,86 | 0% |
| 20 | Adjuvant controls | 0% |
| 21 | Oncosphere controls | 0% |

### EXAMPLE 6: PARTIAL DNA SEQUENCING OF CLONES S2, 48, 95, 119

### Production of pBluescript

The Phagemid pBluescript was generated by phagemid rescue from Lambda-Zap stocks for each cDNA as described in the UNI-ZAP XR Cloning Kit manufacturer's instructions (Stratagene).

### Preparation of single-stranded DNA

Single-stranded DNA was prepared from the plasmid pBluescript (Stratagene) using techniques described by Ausubel *et al*., Eds, "Current Protocols in Molecular Biology", Green Publishing Associates and Wiley Interscience, New York (1987).

### cDNA Sequencing

cDNA's were partially sequenced by chain termination (Sanger F, Nicklen S and Coulson A R, DNA sequencing with chain-terminating inhibitors, Proc Natl Acad Sci USA 14 5463 (1977)) using Sequenase, Version 2.0 (United States Biochemical). The primer was T3 (Albert Einstein College of Medicine, Oligonucleotide Synthesis Unit). Termination reactions were resolved on a 6% polyacrylamide gel and visualised by autoradiography (X-Omat, Kodak).

### Results

The results are presented in Table 5.

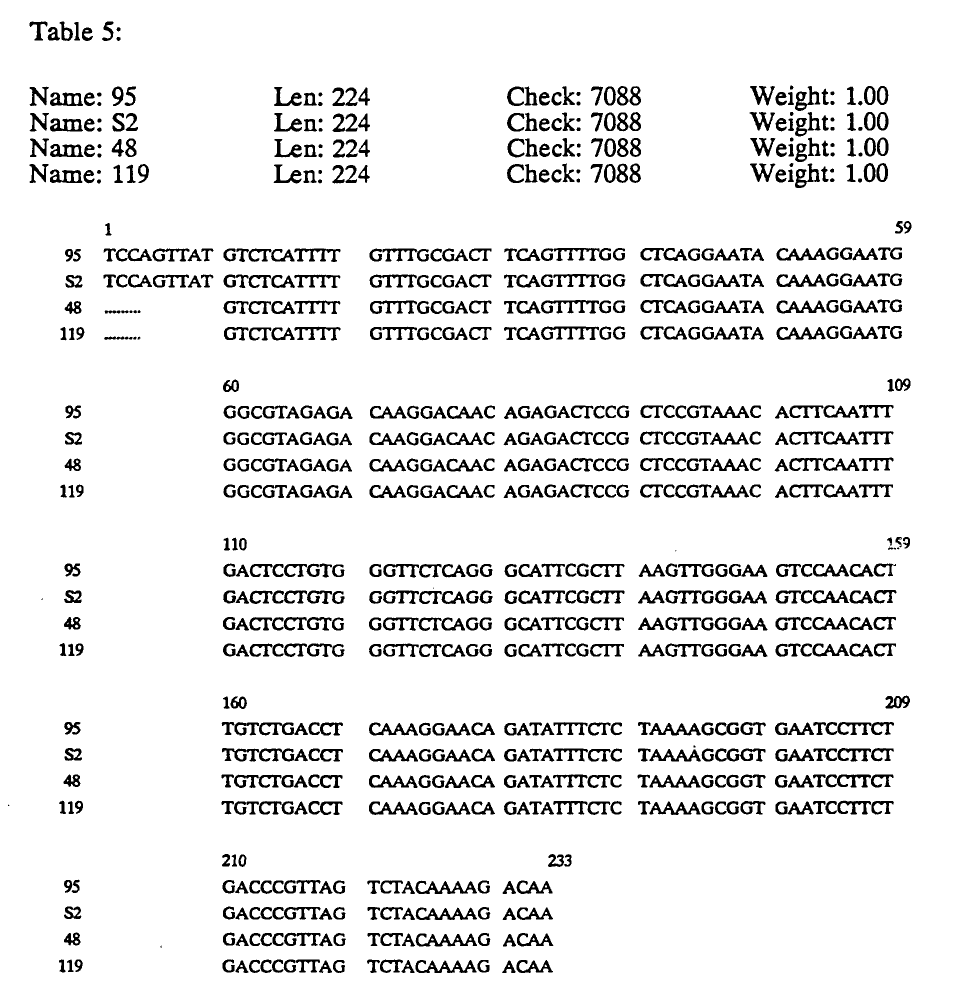

As can be seen from Table 5, each of the clones shown to express antigens protective against *E*. *granulosus* includes an identical 224 bp DNA molecule (nucleotides 10-233 above).

### EXAMPLE 7: DNA SEQUENCING OF CLONE 95

cDNA's were sequenced using the ^{T7}Sequencing Kit (Pharmacia) with CsCl-purified plasmid DNA and oligonucleotide primers derived from the pGEX sequence approximately 30bp up- and down-stream from the *Eco* R1 cloning site. Oligonucleotides were manufactured using a PCR-MATE 391 DNA Synthesizer (Applied Biosystems, Foster City, CA USA) and chemicals from Applied Biosystems. Internal sequence was obtained through the use of forward and reverse oligonucleotide primers designed from the internal cDNA sequence.

The full sequence of clone 95 and the predicted amino acid sequence of the translated protein is shown in Figure 4. Clone 95 cDNA comprises a 715 bp insert (excluding nucleotides derived as part of the cloning strategy into λ ZAP). This cDNA comprises an open reading frame of 461 bp encoding a predicted protein having a molecular weight of 16592 Da.

The following DNA sequence is included in the full sequence of clone 95 and was derived from the strategy used in production of the cDNA and cloned into the λ ZAP XR vector.

At the 5' end of the cDNA:

5' GAATTCGGCACGAG 3'

At the 3' end of the cDNA:

5' AAAAAAAAAAAAAAAAACTCGAG 3'

The sequence of Figure 4 was independently confirmed using the procedure of Example 6.

### INDUSTRIAL APPLICATION

In accordance with the present invention there is provided an antigenic polypeptide together with active peptide fragments and variants of such a polypeptide which are effective in generating a protective immunological response against *E. granulosus* infection in a susceptible host. It has been established that vaccination with this polypeptide and/or its peptide fragments stimulates almost complete immunity against challenge infection with E. *granulosus* eggs. The invention also provides a recombinant method for expression of the antigen by which commercial quantities can be obtained.

### SEQUENCE LISTING

### (1) GENERAL INFORMATION:

(1) APPLICANT: NEW ZEALAND PASTORAL AGRICULTURAL INSTITUTE LIMITED, a company incorporated under the Companies Act 1955 pursuant to the Crown Research Institutes Act 1992 and having its registered office at Peat Marwick Tower, 85 Alexandra Street, Hamilton, New Zealand, THE UNIVERSITY OF MELBOURNE, a body corporate organised and existing under the laws of the State of Victoria, of Grattan Street, Parkville, Victoria 3052, Australia.
(2)TITLE OF INVENTION: Antigens protective against Echinococcus granulosus infection and vaccines containing such antigens.
(3)NUMBER OF SEQUENCES: 4
(4)CORRESPONDENCE ADDRESS:
   (A) ADDRESSEE:A J PARK & SON
   (B) STREET: HUDDART PARKER BUILDING, POST OFFICE SQUARE
   (C) CITY: P O BOX 949, WELLINGTON
   (D) COUNTRY: NEW ZEALAND
(5) COMPUTER READABLE FORM:
   (A) MEDIUM TYPE: 3.5,DS,HD FLOPPY DISC
   (B) COMPUTER: IBM PC COMPATIBLE
   (C) OPERATING SYSTEM: MS-DOS
   (D) SOFTWARE: WORP PERFECT 5.1 FOR WINDOWS
(6)CURRENT APPLICATION DATA:
   (A) APPLICATION NUMBER:
   (B) FILING DATE: 22-FEBRUARY-1993
   (C) CLASSIFICATION:
(7) ATTORNEY/AGENT INFORMATION:
   (A) NAME: BENNETT, MICHAEL R.
(8)TELECOMMUNICATION INFORMATION:
   (A) TELEPHONE: (64 4) 473 8278
   (B) TELEFAX: (64 4) 472 3358
      472 3351

### (2) INFORMATION FOR SEQUENCE ID NO. 1:

(1)SEQUENCE CHARACTERISTICS:
   (A) LENGTH: 224 BASE PAIRS
   (B) TYPE: NUCLEIC ACID
   (C) STRANDEDNESS: SINGLE
   (D) TOPOLOGY: LINEAR
(2)MOLECULE TYPE: cDNA
(3)SEQUENCE DESCRIPTION: SEQ ID NO. 1:

1 GT CTC ATT TTG TTT GCG ACT TCA GTT TTG GCT CAG GAA TAC AAA GGA 47
Leu Ile Leu Phe Ala Thr Ser Val Leu Ala Gln Glu Tyr Lys Gly

48 ATG GGC GTA GAG ACA AGG ACA ACA GAG ACT CCG CTC CGT AAA CAC 92
Met Gly Val Glu Thr Arg Thr Thr Glu Thr Pro Leu Arg Lys His

93 TTC AAT TTG ACT CCT GTG GGT TCT CAG GGC ATT CGC TTA AGT TGG 137
Phe Asn Leu Thr Pro Val Gly Ser Gln Gly Ile Arg Leu Ser Trp

138 GAA GTC CAA CAC TTG TCT GAC CTC AAA GGA ACA GAT ATT TCT CTA 182
Glu Val Gln His Leu Ser Asp Leu Lys Gly Thr Asp Ile Ser Leu

183 AAA GCG GTG AAT CCT TCT GAC CCG TTA GTC TAC AAA AGA CAA 224
Lys Ala Val Asn Pro Ser Asp Pro Leu Val Tyr Lys Arg Gln

### (3) INFORMATION FOR SEQ ID NO. 2:

(1)SEQUENCE CHARACTERISTICS
   (A) LENGTH: 74 AMINO ACIDS
   (B) TYPE: AMINO ACID
   (C) TOPOLOGY: LINEAR
(2)MOLECULE TYPE: PROTEIN
(3)SEQUENCE DESCRIPTION: SEQ ID NO. 2:

1  Leu Ile Leu Phe Ala Thr Ser Val Leu Ala Gln Glu Tyr Lys Gly  15

16  Met Gly Val Glu Thr Arg Thr Thr Glu Thr Pro Leu Arg Lys His  30

31  Phe Asn Leu Thr Pro Val Gly Ser Gln Gly Ile Arg Leu Ser Trp  45

46  Glu Val Gln His Leu Ser Asp Leu Lys Gly Thr Asp Ile Ser Leu  60

61  Lys Ala Val Asn Pro Ser Asp Pro Leu Val Tyr Lys Arg Gln  74

### (4)INFORMATION FOR SEQ ID NO. 3:

(1)SEQUENCE CHARACTERISTICS:
   (A) lENGTH: 715 BASE PAIRS
   (B) TYPE: NUCLEIC ACID
   (C) STRANDEDNESS: SINGLE
   (D) TOPOLOGY: LINEAR
(2) MOLECULE TYPE: cDNA
(3)SEQUENCE DESCRIPTION: SEQ ID NO. 3:

1 TC CAG TTA TGT CTC ATT TTG TTT GCG ACT TCA GTT TTG GCT CAG GAA 47
Gln Leu Cys Leu Ile Leu Phe Ala Thr Ser Val Leu Ala Gln Glu

48 TAC AAA GGA ATG GGC GTA GAG ACA AGG ACA ACA GAG ACT CCG CTC 92
Tyr Lys Gly Met Gly Val Glu Thr Arg Thr Thr Glu Thr Pro Leu

93 CGT AAA CAC TTC AAT TTG ACT CCT GTG GGT TCT CAG GGC ATT CGC 137
Arg Lys His Phe Asn Leu Thr Pro Val Gly Ser Gln Gly Ile Arg

138 TTA AGT TGG GAA GTC CAA CAC TTG TCT GAC CTC AAA GGA ACA GAT 182
Leu Ser Trp Glu Val Gln His Leu Ser Asp Leu Lys Gly Thr Asp

183 ATT TCT CTA AAA GCG GTG AAT CCT TCT GAC CCG TTA GTC TAC AAA 227
Ile Ser Leu Lys Ala Val Asn Pro Ser Asp Pro Leu Val Tyr Lys

228 AGA CAA ACT GCA AAA TTC TCA GAT GGA CAA CTC ACT ATC GGC GAA 272
Arg Gln Thr Ala Lys Phe Ser Asp Gly Gln Leu Thr Ile Gly Glu

273 CTG AAG CCC TCC ACA TTA TAC AAA ATG ACT GTG GAA GCA GTG AAA 317
Leu Lys Pro Ser Thr Leu Tyr Lys Met Thr Val Glu Ala Val Lys

318 GCG AAA AAG ACC ATT TTG GGA TTC ACC GTA GAC ATT GAG ACA CCG 362
Ala Lys Lys Thr Ile Leu Gly Phe Thr Val Asp Ile Glu Thr Pro

363 CGC GCT GGC AAG AAG GAA AGC ACT GTA ATG ACT AGT GGA TCC GCC 407
Arg Ala Gly Lys Lys Glu Ser Thr Val Met Thr Ser Gly Ser Ala

408 TTA ACA TCC GCA ATC GCT GGT TTT GTA TTC AGC TGC ATA GTG GTT 452
Leu Thr Ser Ala Ile Ala Gly Phe Val Phe Ser Cys Ile Val Val

453 GTC CTT ACT TGA ACT CTC ATG TAA GTC AAT GCA AAT TAT CCA CTG 497
Val Leu Thr

498 CTT CTA TAC TGA GTA GCA CGA CCC ATA ACT TGC ATT TTT CAA ATA 542

543 ACT CTT CTT CCA CAT CAG GCT TCC TTG GTG CCG AAG ATG CAC AAA 587

588 TCA CCA TTT ATT TTC GCT TTA TTA ACA TTT GTA TGA CCT CTC ATT 632

633 GTG GAT TAC TCC CGA ATG ACA AAT ACG GGA CTT TGT CAT ATT TGC 677

678 TTC ATT GTT ATC ACC CTT AAT TCC AAT TCA CTG ACT CG 715

### (5) INFORMATION FOR SEQ NO. 4:

(1) SEQUENCE CHARACTERISTICS:
   (A) lENGTH: 153 AMINO ACIDS
   (B) TYPE: AMINO ACID
   (C) TOPOLOGY: LINEAR
(2) MOLECULE TYPE: PROTEIN
(3) SEQUENCE DESCRIPTION: SEQ ID NO. 4:

1 Gln Leu Cys Leu Ile Leu Phe Ala Thr Ser Val Leu Ala Gln Glu 15

16 Tyr Lys Gly Met Gly Val Glu Thr Arg Thr Thr Glu Thr Pro Leu 30

31 Arg Lys His Phe Asn Leu Thr Pro Val Gly Ser Gln Gly Ile Arg 45

46 Leu Ser Trp Glu Val Gln His Leu Ser Asp Leu Lys Gly Thr Asp 60

61 Ile Ser Leu Lys Ala Val Asn Pro Ser Asp Pro Leu Val Tyr Lys 75

76 Arg Gln Thr Ala Lys Phe Ser Asp Gly Gln Leu Thr Ile Gly Glu 90

91 Leu Lys Pro Ser Thr Leu Tyr Lys Met Thr Val Glu Ala Val Lys 105

106 Ala Lys Lys Thr Ile Leu Gly Phe Thr Val Asp Ile Glu Thr Pro 120

121 Arg Ala Gly Lys Lys Glu Ser Thr Val Met Thr Ser Gly Ser Ala 135

136 Leu Thr Ser Ala Ile Ala Gly Phe Val Phe Ser Cys Ile Val Val 150

151 Val Leu Thr 153

## Claims

1. A purified antigenic polypeptide which has a molecular weight in the range of from 23 to 25 kD calculated by SDS-PAGE, which includes the amino acid sequence 
Leu Ile Leu Phe Ala Thr Ser Val Leu Ala Gln Glu Tyr Lys Gly
Met Gly Val Glu Thr Arg Thr Thr Glu Thr Pro Leu Arg Lys His
Phe Asn Leu Thr Pro Val Gly Ser Gln Gly Ile Arg Leu Ser Trp
Glu Val Gln His Leu Ser Asp Leu Lys Gly Thr Asp Ile Ser Leu
Lys Ala Val Asn Pro Ser Asp Pro Leu Val Tyr Lys Arg Gln
and which is capable of generating a protective immunological response to *E. granulosus* infection in a susceptible host;
or a peptide fragment or variant thereof having equivalent protective immunological activity thereto,

2. A peptide fragment according to claim 1 including the amino acid sequence

Leu Ile Leu Phe Ala Thr Ser Val Leu Ala Gln Glu Tyr Lys Gly
Met Gly Val Glu Thr Arg Thr Thr Glu Thr Pro Leu Arg Lys His
Phe Asn Leu Thr Pro Val Gly Ser Gln Gly Ile Arg Leu Ser Trp
Glu Val Gln His Leu Ser Asp Leu Lys Gly Thr Asp Ile Ser Leu
Lys Ala Val Asn Pro Ser Asp Pro Leu Val Tyr Lys Arg Gln .

3. A peptide fragment according to claim 1 consisting of the amino acid sequence

Leu Ile Leu Phe Ala Thr Ser Val Leu Ala Gln Glu Tyr Lys Gly
Met Gly Val Glu Thr Arg Thr Thr Glu Thr Pro Leu Arg Lys His
Phe Asn Leu Thr Pro Val Gly Ser Gln Gly Ile Arg Leu Ser Trp
Glu Val Gln His Leu Ser Asp Leu Lys Gly Thr Asp Ile Ser Leu
Lys Ala Val Asn Pro Ser Asp Pro Leu Val Tyr Lys Arg Gln .

4. A peptide fragment according to claim 1 consisting of the amino acid sequence

Gln Leu Cys Leu Ile Leu Phe Ala Thr Ser Val Leu Ala Gln
Glu Tyr Lys Gly Met Gly Val Glu Thr Arg Thr Thr Glu Thr
Pro Leu Arg Lys His Phe Asn Leu Thr Pro Val Gly Ser Gln
Gly Ile Arg Leu Ser Trp Glu Val Gln His Leu Ser Asp Leu
Lys Gly Thr Asp Ile Ser Leu Lys Ala Val Asn Pro Ser Asp
Pro Leu Val Tyr Lys Arg Gln .

5. A polypeptide or peptide fragment according to claim 1 including the amino acid sequence

Gln Leu Cys Leu Ile Leu Phe Ala Thr Ser Val Leu Ala Gln Glu
Tyr Lys Gly Met Gly Val Glu Thr Arg Thr Thr Glu Thr Pro Leu
Arg Lys His Phe Asn Leu Thr Pro Val Gly Ser Gln Gly Ile Arg
Leu Ser Trp Glu Val Gln His Leu Ser Asp Leu Lys Gly Thr Asp
Ile Ser Leu Lys Ala Val Asn Pro Ser Asp Pro Leu Val Tyr Lys
Arg Gln Thr Ala Lys Phe Ser Asp Gly Gln Leu Thr Ile Gly Glu
Leu Lys Pro Ser Thr Leu Tyr Lys Met Thr Val Glu Ala Val Lys
Ala Lys Lys Thr Ile Leu Gly Phe Thr Val Asp Ile Glu Thr Pro
Arg Ala Gly Lys Lys Glu Ser Thr Val Met Thr Ser Gly Ser Ala
Leu Thr Ser Ala Ile Ala Gly Phe Val Phe Ser Cys Ile Val Val
Val Leu Thr .

6. A peptide fragment according to claim 1 consisting of the amino acid sequence

Gln Leu Cys Leu Ile Leu Phe Ala Thr Ser Val Leu Ala Gln Glu
Tyr Lys Gly Met Gly Val Glu Thr Arg Thr Thr Glu Thr Pro Leu
Arg Lys His Phe Asn Leu Thr Pro Val Gly Ser Gln Gly Ile Arg
Leu Ser Trp Glu Val Gln His Leu Ser Asp Leu Lys Gly Thr Asp
Ile Ser Leu Lys Ala Val Asn Pro Ser Asp Pro Leu Val Tyr Lys
Arg Gln Thr Ala Lys Phe Ser Asp Gly Gln Leu Thr Ile Gly Glu
Leu Lys Pro Ser Thr Leu Tyr Lys Met Thr Val Glu Ala Val Lys
Ala Lys Lys Thr Ile Leu Gly Phe Thr Val Asp Ile Glu Thr Pro
Arg Ala Gly Lys Lys Glu Ser Thr Val Met Thr Ser Gly Ser Ala
Leu Thr Ser Ala Ile Ala Gly Phe Val Phe Ser Cys Ile Val Val
Val Leu Thr .

7. A polypeptide which is a fusion protein comprising a polypeptide or peptide fragment or variant thereof as claimed in any one of claims 1 to 6.

8. A polypeptide as claimed in claim 7 wherein the polypeptide or peptide fragment or variant thereof is fused with the enzyme glutathione s-transferase (E.C 2.5.18).

9. A composition of matter capable of generating a protective immunological response to *E. granulosus* infection in a susceptible host which essentially consists of a component selected from the group consisting of:
(a) the polypeptide of claim 1;
(b) a peptide fragment of (a) having equivalent protective immunological activity thereto; and
(c) a variant of (a) or (b) which has been modified by the insertion, substitution or deletion of one or more amino acids and which has at least equivalent protective immunological activity thereto,

10. A DNA molecule which is selected from the group consisting of:
(a) a nucleotide sequence encoding the antigenic polypeptide of claim 1;
(b) a nucleotide sequence encoding a peptide fragment of the antigenic peptide of (a), which fragment has equivalent protective immunological activity to the polypeptide of (a); and
(c) a nucleotide sequence encoding a variant of the polypeptide of (a) or the peptide of (b) in which the amino acid sequence has been modified by the insertion, substitution or deletion of one or more amino acids, which variant has equivalent protective immunological activity to the polypeptide of (a) or the peptide fragment of (b).

11. A DNA molecule having the DNA sequence

GT CTC ATT TTG TTT GCG ACT TCA GTT TTG
GCT CAG GAA TAC AAA GGA ATG GGC GTA GAG
ACA AGG ACA ACA GAG ACT CCG CTC CGT AAA
CAC TTC AAT TTG ACT CCT GTG GGT TCT CAG
GGC ATT CGC TTA AGT TGG GAA GTC CAA CAC
TTG TCT GAC CTC AAA GGA ACA GAT ATT TCT
CTA AAA GCG GTG AAT CCT TCT GAC CCG TTA
GTC TAC AAA AGA CAA .

12. A DNA molecule having the DNA sequence

CAG TTA TGT CTC ATT TTG TTT GCG ACT TCA GTT TTG GCT CAG GAA TAC AAA
Gln Leu Cys Leu Ile Leu Phe Ala Thr Ser Val Leu Ala Gln Glu Tyr Lys

GGA ATG GGC GTA GAG ACA AGG ACA ACA GAG ACT CCG CTC CGT AAA CAC TTC
Gly Met Gly Val Glu Thr Arg Thr Thr Glu Thr Pro Leu Arg Lys His Phe

AAT TTG ACT CCT GTG GGT TCT CAG GGC ATT CGC TTA AGT TGG GAA GTC CAA
Asn Leu Thr Pro Val Gly Ser Gln Gly Ile Arg Leu Ser Trp Glu Val Gln

CAC TTG TCT GAC CTC AAA GGA ACA GAT ATT TCT CTA AAA GCG GTG AAT CCT
His Leu Ser Asp Leu Lys Gly Thr Asp Ile Ser Leu Lys Ala Val Asn Pro

TCT GAC CCG TTA GTC TAC AAA AGA CAA ACT GCA AAA TTC TCA GAT GGA CAA
Ser Asp Pro Leu Val Tyr Lys Arg Gln Thr Ala Lys Phe Ser Asp Gly Gln

CTC ACT ATC GGC GAA CTG AAG CCC TCC ACA TTA TAC AAA ATG ACT GTG GAA
Leu Thr Ile Gly Glu Leu Lys Pro Ser Thr Leu Tyr Lys Met Thr Val Glu

GCA GTG AAA GCG AAA AAG ACC ATT TTG GGA TTC ACC GTA GAC ATT GAG ACA
Ala Val Lys Ala Lys Lys Thr Ile Leu Gly Phe Thr Val Asp Ile Glu Thr

CCG CGC GCT GGC AAG AAG GAA AGC ACT GTA ATG ACT AGT GGA TCC GCC TTA
Pro Arg Ala Gly Lys Lys Glu Ser Thr Val Met Thr Ser Gly Ser Ala Leu

ACA TCC GCA ATC GCT GGT TTT GTA TTC AGC TGC ATA GTG GTT GTC CTT ACT
Thr Ser Ala Ile Ala Gly Phe Val Phe Ser Cys Ile Val Val Val Leu Thr

13. A DNA molecule having the DNA sequence

TC CAG TTA TGT CTC ATT TTG TTT GCG ACT TCA GTT TTG GCT CAG GAA TAC AAA
Gln Leu Cys Leu Ile Leu Phe Ala Thr Ser Val Leu Ala Gln Glu Tyr Lys

GGA ATG GGC GTA GAG ACA AGG ACA ACA GAG ACT CCG CTC CGT AAA CAC TTC
Gly Met Gly Val Glu Thr Arg Thr Thr Glu Thr Pro Leu Arg Lys His Phe

AAT TTG ACT CCT GTG GGT TCT CAG GGC ATT CGC TTA AGT TGG GAA GTC CAA
Asn Leu Thr Pro Val Gly Ser Gln Gly Ile Arg Leu Ser Trp Glu Val Gln

CAC TTG TCT GAC CTC AAA GGA ACA GAT ATT TCT CTA AAA GCG GTG AAT CCT
His Leu Ser Asp Leu Lys Gly Thr Asp Ile Ser Leu Lys Ala Val Asn Pro

TCT GAC CCG TTA GTC TAC AAA AGA CAA ACT GCA AAA TTC TCA GAT GGA CAA
Ser Asp Pro Leu Val Tyr Lys Arg Gln Thr Ala Lys Phe Ser Asp Gly Gln

CTC ACT ATC GGC GAA CTG AAG CCC TCC ACA TTA TAC AAA ATG ACT GTG GAA
Leu Thr Ile Gly Glu Leu Lys Pro Ser Thr Leu Tyr Lys Met Thr Val Glu

GCA GTG AAA GCG AAA AAG ACC ATT TTG GGA TTC ACC GTA GAC ATT GAG ACA
Ala Val Lys Ala Lys Lys Thr Ile Leu Gly Phe Thr Val Asp Ile Glu Thr

CCG CGC GCT GGC AAG AAG GAA AGC ACT GTA ATG ACT AGT GGA TCC GCC TTA
Pro Arg Ala Gly Lys Lys Glu Ser Thr Val Met Thr Ser Gly Ser Ala Leu

ACA TCC GCA ATC GCT GGT TTT GTA TTC AGC TGC ATA GTG GTT GTC CTT ACT
Thr Ser Ala Ile Ala Gly Phe Val Phe Ser Cys Ile Val Val Val Leu Thr

TGAACTCTCATGTAAGTCAATGCAAATTATCCACTGCTTCTATACTGAGTAGCACGACCCATAACTT

GCATTTTTCAAATAACTCTTCTTCCACATCAGGCTTCCTTGGTGCCGAAGATGCACAAATCACCATT

TATTTTCGCTTTATTAACATTTGTATGACCTCTCATTGTGGATTACTCCCGAATGACAAATACGGGA

CTTTGTCATATTTGCTTCATTGTTATCACCCTTAATTCCAATTCACTGACTCG

14. A DNA molecule as claimed in any one of claims 10 to 13 which is cDNA.

15. A recombinant expression vector which contains a DNA molecule as claimed in any one of claims 10 to 14.

16. A host cell transformed with a vector as claimed in claim 15 and capable of expressing the polypeptide or peptide fragment or variant thereof which is encoded.

17. A host cell as claimed in claim 16 which is a prokaryote.

18. A host cell as claimed in claim 17 wherein the prokaryote host is *E. coli.*

19. A host cell as claimed in claim 16 which is a eukaryote.

20. A method of producing an antigenic polypeptide or a peptide fragment or variant thereof, which comprises culturing a cell as claimed in any one of claims 16 to 19 and recovering the expressed product.

21. A vaccine against *E. granulosus* infection comprising an immunologically-effective amount of a polypeptide, peptide fragment or variant as claimed in any one of claims 1 to 8 in combination with a pharmaceutically acceptable adjuvant, carrier or diluent therefor.

22. A vaccine as claimed in claim 21 which further includes an effective amount of one or more *E. granulosus* polypeptide(s) having a molecular weight determined by SDS-PAGE selected from 30 kD, 34 kD and 40 kD, or of one or more fragments or variants of said polypeptides having equivalent immunological activity thereto.

23. A recombinant viral vaccine which includes nucleic acid encoding an antigenic polypeptide or peptide fragment or variant thereof as claimed in claim 1 and which is capable of expressing said encoded polypeptide or peptide fragment or variant.

24. A polypeptide, peptide fragment or variant as claimed in any one of claims 1 to 8 for use in a method of protecting a host susceptible to infection by an *Echinococcus* or *Taeniid* parasite against such infection comprising administering to a said host an immunologically effective amount of a said polypeptide, peptide fragment or variant.

25. The use of a polypeptide, peptide fragment or variant as claimed in any one of claims 1 to 8 in the manufacture of a medicament for protecting a host susceptible to infection by an *Echinococcus* or *Taeniid* parasite against such infection.

26. The use of claim 25 wherein said parasite is *E. granulosus*.

27. The use of claim 25 wherein said parasite is *E. multilocularis, E. vogelii, T. ovis, T. saginata, T. solium, T. multiceps or T. hydatigena.*

28. A purified antibody or binding fragment thereof specific for an antigenic polypeptide, peptide fragment or variant as claimed in claim 1.

29. The use of an antibody or binding fragment thereof as claimed in claim 28 to identify a protective antigen of an *Echinococcus* or *Taeniid* parasite other than *E.granulosus.*

30. An optionally-labelled DNA molecule comprising part or all of the nucleotide sequence of Figure 4 suitable for use as a probe for identifying nucleic acid coding for a protective antigen of an *Echinococcus* or *Taeniid* parasite other than *E. granulosus.*

31. The use of a DNA molecule as claimed in claim 30 to identify DNA encoding a protective antigen of an *Echinococcus* or *Taeniid* parasite other than *E. granulosus.*

32. The use of claim 29 or claim 31 wherein the parasite other than *E. granulosus* is *E. multilocularis*, *E. vogelii, T. ovis. T. saginata, T. solium, T. multiceps or T. hydatigena.*

33. A method of identifying a protective antigen of an *Echinococcus* or *Taeniid* parasite other than *E. granulosus* which comprises the step of identifying a gene of said parasite having a nucleotide sequence which is at least substantially homologous to part or all of the nucleotide sequence of Figure 4.

## Patentansprüche

1. Gereinigtes antigenisches Polypeptid mit einem Molekulargewicht im Bereich von 23 bis 25 kD, berechnet durch SDS-PAGE, das die Aminosäuresequenz 
Leu Ile Leu Phe Ala Thr Ser Val Leu Ala Gln Glu Tyr Lys Gly
Met Gly Val Glu Thr Arg Thr Thr Glu Thr Pro Leu Arg Lys His
Phe Asn Leu Thr Pro Val Gly Ser Gln Gly Ile Arg Leu Ser Trp
Glu Val Gln His Leu Ser Asp Leu Lys Gly Thr Asp Ile Ser Leu
Lys Ala Val Asn Pro Ser Asp Pro Leu Val Tyr Lys Arg Gln
 umfasst und fähig ist, in einem anfälligen Wirt eine immunologische Schutzreaktion auf E. granulosus-Infektion hervorzurufen;
oder ein Peptidfragment oder eine Variante davon mit dazu äquivalenter immunologischer Schutzwirkung dagegen.

2. Peptidfragment nach Anspruch 1, das die Aminosäuresequenz 
Leu Ile Leu Phe Ala Thr Ser Val Leu Ala Gln Glu Tyr Lys Gly
Met Gly Val Glu Thr Arg Thr Thr Glu Thr Pro Leu Arg Lys His
Phe Asn Leu Thr Pro Val Gly Ser Gln Gly Ile Arg Leu Ser Trp
Glu Val Gln His Leu Ser Asp Leu Lys Gly Thr Asp Ile Ser Leu
Lys Ala Val Asn Pro Ser Asp Pro Leu Val Tyr Lys Arg Gln .
 umfasst.

3. Peptidfragment nach Anspruch 1, das aus der Aminosäuresequenz 
. Leu Ile Leu Phe Ala Thr Ser Val Leu Ala Gln Glu Tyr Lys Gly
Met Gly Val Glu Thr Arg Thr Thr Glu Thr Pro Leu Arg Lys His
Phe Asn Leu Thr Pro Val Gly Ser Gln Gly Ile Arg Leu Ser Trp
Glu Val Gln His Leu Ser Asp Leu Lys Gly Thr Asp Ile Ser Leu
Lys Ala Val Asn Pro Ser Asp Pro Leu Val Tyr Lys Arg Gln .
 besteht.

4. Peptidfragment nach Ansoruch 1, das aus der Aminosäuresequenz 
Gln Leu Cys Leu Ile Leu Phe Ala Thr Ser Val Leu Ala Gln
Glu Tyr Lys Gly Met Gly Val Glu Thr Arg Thr Thr Glu Thr
Pro Leu Arg Lys His Phe Asn Leu Thr Pro Val Gly Ser Gln
Gly Ile Arg Leu Ser Trp Glu Val Gln His Leu Ser Asp Leu
Lys Gly Thr Asp Ile Ser Leu Lys Ala Val Asn Pro Ser Asp
Pro Leu Val Tyr Lys Arg Gln .
 besteht.

5. Polypeptid oder Peptidfragment nach Anspruch 1, das die Aminosäuresequenz 
Gln Leu Cys Leu Ile Leu Phe Ala Thr Ser Val Leu Ala Gln Glu
Tyr Lys Gly Met Gly Val Glu Thr Arg Thr Thr Glu Thr Pro Leu
Arg Lys His Phe Asn Leu Thr Pro Val Gly Ser Gln Gly Ile Arg
Leu Ser Trp Glu Val Gln His Leu Ser Asp Leu Lys Gly Thr Asp
Ile Ser Leu Lys Ala Val Asn Pro Ser Asp Pro Leu Val Tyr Lys
Arg Gln Thr Ala Lys Phe Ser Asp Gly Gln Leu Thr Ile Gly Glu
Leu Lys Pro Ser Thr Leu Tyr Lys Met Thr Val Glu Ala Val Lys
Ala Lys Lys Thr Ile Leu Gly Phe Thr Val Asp Ile Glu Thr Pro
Arg Ala Gly Lys Lys Glu Ser Thr Val Met Thr Ser Gly Ser Ala
Leu Thr Ser Ala Ile Ala Gly Phe Val Phe Ser Cys Ile Val Val
Val Leu Thr .
 enthält.

6. Peptidfragment nach Anspruch 1, das aus der Aminosäureseguenz 
Gln Leu Cys Leu Ile Leu Phe Ala Thr Ser Val Leu Ala Gln Glu
Tyr Lys Gly Met Gly Val Glu Thr Arg Thr Thr Glu Thr Pro Leu
Arg Lys His Phe Asn Leu Thr Pro Val Gly Ser Gln Gly Ile Arg
Leu Ser Trp Glu Val Gln His Leu Ser Asp Leu Lys Gly Thr Asp
Ile Ser Leu Lys Ala Val Asn Pro Ser Asp Pro Leu Val Tyr Lys
Arg Gln Thr Ala Lys Phe Ser Asp Gly Gln Leu Thr Ile Gly Glu
Leu Lys Pro Ser Thr Leu Tyr Lys Met Thr Val Glu Ala Val Lys
Ala Lys Lys Thr Ile Leu Gly Phe Thr Val Asp Ile Glu Thr Pro
 
Arg Ala Gly Lys Lys Glu Ser Thr Val Met Thr Ser Gly Ser Ala
Leu Thr Ser Ala Ile Ala Gly Phe Val Phe Ser Cys Ile Val Val
Val Leu Thr .
 besteht.

7. Polypeptid, das ein Fusionsprotein ist, das ein Polypeptid oder Peptidfragment oder eine Variante davon nach einem der Ansprüche 1 bis 6 umfasst.

8. Polypeptid nach Anspruch 7, worin das Polypeptid oder Peptidfragment oder die Variante davon mit dem Enzym Glutathion-s-transferase (E.C 2.5.18) fusioniert ist.

9. Materialzusammensetzung, die fähig ist, in einem anfälligen Wirt eine immunologische Schutzreaktion auf E. granulosus-Infektion hervorzurufen und die im Wesentlichen aus einer Komponente besteht, die aus der Gruppe ausgewählt ist, die besteht aus:
(a) dem Polypeptid nach Anspruch 1;
(b) einem Peptidfragment von (a) mit dazu äquivalenter immunologischer Schutzwirkung dagegen; und
(c) einer Variante von (a) oder (b), die durch die Insertion, Substitution oder Deletion einer oder mehrerer Aminosäuren modifiziert worden ist und die zumindest dazu äquivalente immunologische Schutzwirkung dagegen aufweist.

10. DNA-Molekül, das aus der Gruppe ausgewählt ist, die besteht aus:
(a) einer Nukleotidsequenz, die für das antigenische Polypeptid nach Anspruch 1 kodiert;
(b) einer Nukleotidsequenz, die für ein Peptidfragment des antigenischen Peptids von (a) kodiert, wobei das Fragment zum Polypeptid von (a) äquivalente immunologische Schutzwirkung aufweist; und
(c) eine Nukleotidsequenz, die für eine Variante des Polypeptids von (a) oder des Peptids von (b) kodiert, worin die Aminosäuresequenz durch die Insertion, Substitution oder Deletion einer oder mehrerer Aminosäuren modifiziert worden ist, wobei die Variante zum Polypeptid von (a) oder zum Peptidfragment von (b) äquivalente immunologische Schutzwirkung aufweist.

11. DNA-Molekül mit der DNA-Sequenz:

GT CTC ATT TTG TTT GCG ACT TCA GTT TTG
GCT CAG GAA TAC AAA GGA ATG GGC GTA GAG
ACA AGG ACA ACA GAG ACT CCG CTC CGT AAA
CAC TTC AAT TTG ACT CCT GTG GGT TCT CAG
GGC ATT CGC TTA AGT TGG GAA GTC CAA CAC
TTG TCT GAC CTC AAA GGA ACA GAT ATT TCT
CTA AAA GCG GTG AAT CCT TCT GAC CCG TTA
GTC TAC AAA AGA CAA .

12. DNA-Molekül mit der DNA-Sequenz

CAG TTA TGT CTC ATT TTG TTT GCG ACT TCA GTT TTG GCT CAG GAA TAC AAA
Gln Leu Cys Leu Ile Leu Phe Ala Thr Ser Val Leu Ala Gln Glu Tyr Lys

GGA ATG GGC GTA GAG ACA AGG ACA ACA GAG ACT CCG CTC CGT AAA CAC TTC
Gly Met Gly Val Glu Thr Arg Thr Thr Glu Thr Pro Leu Arg Lys His Phe

AAT TTG ACT CCT GTG GGT TCT CAG GGC ATT CGC TTA AGT TGG GAA GTC CAA
Asn Leu Thr Pro Val Gly Ser Gln Gly Ile Arg Leu Ser Trp Glu Val Gln

CAC TTG TCT GAC CTC AAA GGA ACA GAT ATT TCT CTA AAA GCG GTG AAT CCT
His Leu Ser Asp Leu Lys Gly Thr Asp Ile Ser Leu Lys Ala Val Asn Pro

TCT GAC CCG TTA GTC TAC AAA AGA CAA ACT GCA AAA TTC TCA GAT GGA CAA
Ser Asp Pro Leu Val Tyr Lys Arg Gln Thr Ala Lys Phe Ser Asp Gly Gln

CTC ACT ATC GGC GAA CTG AAG CCC TCC ACA TTA TAC AAA ATG ACT GTG GAA
Leu Thr Ile Gly Glu Leu Lys Pro Ser Thr Leu Tyr Lys Met Thr Val Glu

GCA GTG AAA GCG AAA AAG ACC ATT TTG GGA TTC ACC GTA GAC ATT GAG ACA
Ala Val Lys Ala Lys Lys Thr Ile Leu Gly Phe Thr Val Asp Ile Glu Thr

CCG CGC GCT GGC AAG AAG GAA AGC ACT GTA ATG ACT AGT GGA TCC GCC TTA
Pro Arg Ala Gly Lys Lys Glu Ser Thr Val Met Thr Ser Gly Ser Ala Leu

ACA TCC GCA ATC GCT GGT TTT GTA TTC AGC TGC ATA GTG GTT GTC CTT ACT
Thr Ser Ala Ile Ala Gly Phe Val Phe Ser Cys Ile Val Val Val Leu Thr

13. DNA-Molekül mit der DNA-Sequenz

TC CAG TTA TGT CTC ATT TTG TTT GCG ACT TCA GTT TTG GCT CAG GAA TAC AAA
Gln Leu Cys Leu Ile Leu Phe Ala Thr Ser Val Leu Ala Gln Glu Tyr Lys

GGA ATG GGC GTA GAG ACA AGG ACA ACA GAG ACT CCG CTC CGT AAA CAC TTC
Gly Met Gly Val Glu Thr Arg Thr Thr Glu Thr Pro Leu Arg Lys His Phe

AAT TTG ACT CCT GTG GGT TCT CAG GGC ATT CGC TTA AGT TGG GAA GTC CAA
Asn Leu Thr Pro Val Gly Ser Gln Gly Ile Arg Leu Ser Trp Glu Val Gln

CAC TTG TCT GAC CTC AAA GGA ACA GAT ATT TCT CTA AAA GCG GTG AAT CCT
His Leu Ser Asp Leu Lys Gly Thr Asp Ile Ser Leu Lys Ala Val Asn Pro

TCT GAC CCG TTA GTC TAC AAA AGA CAA ACT GCA AAA TTC TCA GAT GGA CAA
Ser Asp Pro Leu Val Tyr Lys Arg Gln Thr Ala Lys Phe Ser Asp Gly Gln

CTC ACT ATC GGC GAA CTG AAG CCC TCC ACA TTA TAC AAA ATG ACT GTG GAA
Leu Thr Ile Gly Glu Leu Lys Pro Ser Thr Leu Tyr Lys Met Thr Val Glu

GCA GTG AAA GCG AAA AAG ACC ATT TTG GGA TTC ACC GTA GAC ATT GAG ACA
Ala Val Lys Ala Lys Lys Thr Ile Leu Gly Phe Thr Val Asp Ile Glu Thr

CCG CGC GCT GGC AAG AAG GAA AGC ACT GTA ATG ACT AGT GGA TCC GCC TTA
Pro Arg Ala Gly Lys Lys Glu Ser Thr Val Met Thr Ser Gly Ser Ala Leu

ACA TCC GCA ATC GCT GGT TTT GTA TTC AGC TGC ATA GTG GTT GTC CTT ACT
Thr Ser Ala Ile Ala Gly Phe Val Phe Ser Cys Ile Val Val Val Leu Thr

TGAACTCTCATGTAAGTCAATGCAAATTATCCACTGCTTCTATACTGAGTAGCACGACCCATAACTT

GCATTTTTCAAATAACTCTTCTTCCACATCAGGCTTCCTTGGTGCCG[?][?]GATCCACAAAT[?]ACCATT

TATTTTCGCTTTATTAACATTTGTATGACCTCTCATTGTGGATTACTCCCGAATGACAAATACGGGA

CTTTGTCATATTTGCTTCATTGTTATCACCCTTAATTCCAATTCACTGACTCG

14. DNA-Molekül nach einem der Ansprüche 10 bis 13, das cDNA ist.

15. Rekombinanter Expressionsvektor, der ein DNA-Molekü nach einem der Ansprüche 10 bis 14 enthält.

16. Wirtszelle, die mit einem Vektor nach Anspruch 15 transformiert ist und fähig ist, das Polypeptid oder Peptidfragment oder die Variante davon zu exprimieren, für das/die kodiert wird.

17. Wirtszelle nach Anspruch 16, die ein Prokaryot ist.

18. Wirtszelle nach Anspruch 17, worin der prokaroytische Wirt E. coli ist.

19. Wirtszelle nach Anspruch 16, die ein Eukaryot ist.

20. Verfahren zur Herstellung eine antigenischen Polypeptids oder eines Peptidfragments oder einer Variante davon, welches das Kultivieren einer Zelle nach einem der Ansprüche 16 bis 19 und das Gewinnen des exprimierten Produktes umfasst.

21. Impfstoff gegen E. granulosus-Infektion, umfassend eine immunologisch wirksame Menge eines Polypeptids, eines Peptidfragments oder einer Variante nach einem der Ansprüche 1 bis 8 in Kombination mit einem pharmazeutisch annehmbaren Adjuvans, Träger oder Verdünner dafür.

22. Impfstoff nach Anspruch 21, der weiters eine wirksame Menge eines oder mehrerer E. granulosus-Peptids bzw. Peptide mit einem durch SDS-PAGE bestimmten Molekulargewicht, das aus 30 kD, 34 kD und 40 kD ausgewählt ist, oder ein oder mehrere Fragmente oder Varianten der Polypeptide mit dazu äquivalenter immunologischer Aktivität dagegen enthält.

23. Rekombinanter viraler Impfstoff, der Nukleinsäure umfasst, die für ein antigenisches Polypeptid oder Peptidfragment oder eine Variante davon nach Anspruch 1 kodiert, die fähig ist, das/die kodierte Polypeptid oder Peptidfragment oder Variante zu exprimieren.

24. Polypeptid, Peptidfragment oder Variante nach einem der Ansprüche 1 bis 8 zur Verwendung bei einem Verfahren zum Schutz eines Wirts, der für Infektion durch einen Echinococcus- oder Taeniid-Parasiten anfällig ist, gegen eine solche Infektion, umfassend die Verabreichung einer immunologisch wirksamen Menge des Polypeptids, des Peptidfragments oder der Variante an den Wirt.

25. Verwendung eines Polypeptids, eines Peptidfragments oder einer Variante nach einem der Ansprüche 1 bis 8 bei der Herstellung eines Medikaments zum Schutz eines für Infektion durch einen Echinococcus- oder Taeniid-Parasiten anfälligen Wirts gegen derartige Infektion.

26. Verwendung nach Anspruch 25, worin der Parasit E. granulosus ist.

27. Verwendung nach Anspruch 25, worin der Parasit E. multilocularis, E. vogelii, T. ovis, T. saginata, T. solium, T. multiceps oder T. hydatigena ist.

28. Gereinigter Antikörper oder Bindungsfragment davon, der/das für ein antigenisches Polypeptid, Peptidfragment oder eine Variante nach Anspruch 1 spezifisch ist.

29. Verwendung eines Antikörpers oder Bindungsfragments davon nach Anspruch 28 zum Identifizieren eines schützenden Antigens eines Echinococcus- oder Taeniid-Parasiten mit Ausnahme von E. granulosus.

30. Gegebenenfalls markiertes DNA-Molekül, das einen Teil der oder die gesamte Nukleotidsequenz von Fig. 4 umfasst, die zur Verwendung als Sonde zum Identifizieren von Nukleinsäure geeignet ist, die für ein schützendes Antigen eines Echinococcus- oder Taeniid-Parasiten mit Ausnahme von E. granulosus kodiert.

31. Verwendung eines DNA-Moleküls nach Anspruch 30 zum Identifizieren von DNA, die für ein schützendes Antigen eines Echinococcus- oder Taeniid-Parasiten mit Ausnahme von E. granulosus kodiert.

32. Verwendung nach Anspruch 29 oder 31, worin der Parasit mit Ausnahme von E. granulosus E. multilocularis, E. vogelii, T. ovis, T. saginata, T. solium, T. multiceps oder T. hydatigena ist.

33. Verfahren zum Identifizieren eines schützenden Antigens eines Echinococcus- oder Taeniid-Parasiten mit Ausnahme von E. granulosus, welches den Schritt des Identifizierens eines Gens des Parasiten mit einer Nukleotidsequenz umfasst, die zumindest im Wesentlichen homolog mit einem Teil der oder der gesamten Nukleotidsequenz von Fig. 4 ist.

## Revendications

1. Polypeptide antigénique purifié qui a un poids moléculaire dans la gamme de 23 à 25 kD calculé par SDS-PAGE, qui comprend la séquence d'acides aminés 
Leu Ile Leu Phe Ala Thr Ser Val Leu Ala Gln Glu Tyr Lys Gly
Met Gly Val Glu Thr Arg Thr Thr Gly Thr Pro Leu Arg Lys His
Phe Asn Leu Thr Pro Val Gly Ser Gln Gly Ile Arg Leu Ser Trp
Glu Val Gln His Leu Ser Asp Leu Lys Gly Thr Asp Ile Ser Leu
Lys Ala Val Asn Pro Ser Asp Pro Leu Val Tyr Lys Arg Gln
et qui est capable de générer une réponse immunologique protectrice à une infection par *E.granulosus* chez un hôte sensible ;
ou son fragment peptidique ou variant ayant une activité immunologique protectrice équivalente.

2. Fragment de peptide selon la revendication 1, comprenant la séquence d'acides aminés

Leu Ile Leu Phe Ala Thr Ser Val Leu Ala Gln Glu Tyr Lys Gly
Met Gly Val Glu Thr Arg Thr Thr Glu Thr Pro Leu Arg Lys His
Phe Asn Leu Thr Pro Val Gly Ser Gln Gly Ile Arg Leu Ser Trp
Glu Val Gln His Leu Ser Asp Leu Lys Gly Thr Asp Ile Ser Leu
Lys Ala Val Asn Pro Ser Asp Pro Leu Val Tyr Lys Arg Gln.

3. Fragment de peptide selon la revendication 1, consistant en la sequence d'acides aminés

Leu Ile Leu Phe Ala Thr Ser Val Leu Ala Gln Glu Tyr Lys Gly
Met Gly Val Glu Thr Arg Thr Thr Glu Thr Pro Leu Arg Lys His
Phe Asn Leu Thr Pro Val Gly Ser Gln Gly Ile Arg Leu Ser Trp
Glu Val Gln His Leu Ser Asp Leu Lys Gly Thr Asp Ile Ser Leu
Lys Ala Val Asn Pro Ser Asp Pro Leu Val Tyr Lys Arg Gln.

4. Fragment de peptide selon la revendication 1, consistant en la sequence d'acides aminés

Gln Leu Cys Leu Ile Leu Phe Ala Thr Ser Val Leu Ala Gln
Glu Tyr Lys Gly Met Gly Val Glu Thr Arg Thr Thr Glu Thr
Pro Leu Arg Lys His Phe Asn Leu Thr Pro Val Gly Ser Gln
Gly Ile Arg Leu Ser Trp Glu Val Gln His Leu Ser Asp Leu
Lys Gly Thr Asp Ile Ser Leu Lys Ala Val Asn Pro Ser Asp
Pro Leu Val Tyr Lys Arg Gln.

5. Polypeptide ou fragment de polypeptide selon la revendication 1, comprenant la'séquence d'acides aminés

Gln Leu Cys Leu Ile Leu Phe Ala Thr Ser Val Leu Ala Gln Glu
Tyr Lys Gly Met Gly Val Glu Thr Arg Thr Thr Glu Thr Pro Leu
Arg Lys His Phe Asn Leu Thr Pro Val Gly Ser Gln Gly Ile Arg
Leu Ser Trp Glu Val Gln His Leu Ser Asp Leu Lys Gly Thr Asp
Ile Ser Leu Lys Ala Val Asn Pro Ser Asp Pro Leu Val Tyr Lys
Arg Gln Thr Ala Lys Phe Ser Asp Gly Gln Leu Thr Ile Gly Glu
Leu Lys Pro Ser Thr Leu Tyr Lys met Thr Val Glu Ala Val Lys
Ala Lys Lys Thr Ile Leu Gly Phe Thr Val Asp Ile Glu Thr Pro
Arg Ala Gly Lys Lys Glu Ser Thr Val Met Thr Ser Gly Ser Ala
Leu Thr Ser Ala Ile Ala Gly Phe Val Phe Ser Cys Ile Val Val
Val Leu Thr.

6. Fragment de peptide selon la revendication 1 consistant en la séquence d'acides aminés

Gln Leu Cys Leu Ile Leu Phe Ala Thr Ser Val Leu Ala Gln Glu
Tyr Lys Gly Met Gly Val Glu Thr Arg Thr Thr Glu Thr Pro Leu
Arg Lys His Phe Asn Leu Thr Pro Val Gly Ser Gln Gly Ile Arg
Leu Ser Trp Glu Val Gln His Leu Ser Asp Leu Lys Gly Thr Asp
Ile Ser Leu Lys Ala Val Asn Pro Ser Asp Pro Leu Val Tyr Lys
Arg Gln Thr Ala Lys Phe Ser Asp Gly Gln Leu Thr Ile Gly Glu
Leu Lys Pro Ser Thr Leu Tyr Lys Met Thr Val Glu Ala Val Lys
Ala Lys Lys Thr Ile Leu Gly Phe Thr Val Asp Ile Glu Thr Pro

Arg Ala Gly Lys Lys Glu Ser Thr Val Met Thr Ser Gly Ser Ala
Leu Thr Ser Ala Ile Ala Gly Phe Val Phe Ser Cys Ile Val Val
Val Leu Thr

7. Polypeptide qui est une protéine de fusion comprenant un polypeptide ou fragment de peptide ou son variant selon l'une quelconque des revendications 1 à 6.

8. Polypeptide selon la revendication 7 où le polypeptide ou le fragment de peptide ou son variant est fusionné à l'enzyme glutathione s-transférase (E.C 2.5.18).

9. Composition de matières capable de générer une réponse immunologique protectrice à une infection par *E.granulosus* chez un hôte sensible, qui consiste essentiellement en un composant sélectionné dans le groupe consistant en :
(a) le polypeptide de la revendication 1 ;
(b) un fragment de peptide de (a) ayant une activité immunologique protectrice équivalente à celui-ci ; et
(c) un variant de (a) ou (b) qui a été modifié par l'insertion, la substitution ou la délétion d'un ou plusieurs acides aminés et qui a une activité immunologique protectrice au moins équivalente à eux.

10. Molécule d'ADN qui est sélectionnée dans le groupe consistant en :
(a) une séquence de nucléotides codant pour le polypeptide antigénique de la revendication 1 ;
(b) une séquence de nucléotides codant pour un fragment de peptide du peptide antigénique de (a), lequel fragment a une activité immunologique protectrice équivalente au polypeptide de (a) ; et
(c) une séquence de nucléotides codant pour un variant du polypeptide de (a) ou du peptide de (b), où la séquence d'acides aminés a été modifiée par l'insertion, la substitution ou la délétion d'un ou plusieurs acides aminés, lequel variant a une activité immunologique protectrice équivalente au polypeptide de (a) ou au fragment de peptide de (b).

11. Molécule d'ADN ayant la séquence d'ADN

GT CTC ATT TTG TTT GCG ACT TCA GTT TTG
GCT CAG GAA TAC AAA GGA ATG GGC GTA GAG
ACA AGG ACA ACA GAG ACT CCG CTC CGT AAA
CAC TTC AAT TTG ACT CCT GTG GGT TCT CAG
GGC ATT CGC TTA AGT TGG GAA GTC CAA CAC
TTC TCT GAC CTC AAA GGA ACA GAT ATT TCT
CTA AAA GCG GTG AAT CCT TCT GAC CCG TTA
GTC TAC AAA AGA CAA

12. Molécule d'ADN ayant la séquence d'ADN

CAG TTA TGT CTC ATT TTG TTT GCG ACT TCA GTT TTG GCT CAG GAA TAC AAA
Gln Leu Cys Leu Ile Leu Phe Ala Thr Ser Val Leu Ala Gln Glu Tyr Lys

GGA ATG GGC GTA GAG ACA AGG ACA ACA GAG ACT CCG CTC CGT AAA CAC TTC
Gly Met Gly Val Glu Thr Arg Thr Thr Glu Thr Pro Leu Arg Lys His Phe

AAT TTG ACT CCT GTG GGT TCT CAG GGC ATT CGC TTA AGT TGG GAA GTC CAA
Asn Leu Thr Pro Val Gly Ser Gln Gly Ile Arg Leu Ser Trp Glu Val Gln

CAC TTG TCT GAC CTC AAA GGA ACA GAT ATT TCT CTA AAA GCG GTG AAT CCT
His Leu Ser Asp Leu Lys Gly Thr Asp Ile Ser Leu Lys Ala Val Asn Pro

TCT GAC CCG TTA GTC TAC AAA AGA CAA ACT GCA AAA TTC TCA GAT GGA CAA
Ser Asp Pro Leu Val Tyr Lys Arg Gln Thr Ala Lys Phe Ser Asp Gly Gln

CTC ACT ATC GGC GAA CTG AAG CCC TCC ACA TTA TAC AAA ATG ACT GTG GAA
Leu Thr Ile Gly Glu Leu Lys Pro Ser Thr Leu Tyr Lys Met Thr Val Glu

GCA GTG AAA GCG AAA AAG ACC ATT TTG GGA TTC ACC GTA GAC ATT GAG ACA
Ala Val Lys Ala Lys Lys Thr Ile Leu Gly Phe Thr Val Asp Ile Glu Thr

CCG CGC GCT GGC AAG AAG GAA AGC ACT GTA ATG ACT AGT GGA TCC GCC TTA
Pro Arg Ala Gly Lys Lys Glu Ser Thr Val Met Thr Ser Gly Ser Ala Leu

ACA TCC GCA ATC GCT GGT TTT GTA TTC AGC TGC ATA GTG GTT GTC CTT ACT
Thr Ser Ala Ile Ala Gly Phe Val Phe Ser Cys Ile Val Val Val Leu Thr

13. Molécule d'ADN ayant la séquence d'ADN

TC CAG TTA TGT CTC ATT TTG TTT GCG ACT TCA GTT TTG GCT CAG GAA TAC AAA
Gln Leu Cys Leu Ile Leu Phe Ala Thr Ser Val Leu Ala Gln Glu Tyr Lys

GGA ATG GGC GTA GAG ACA AGG ACA ACA GAG ACT CCG CTC CGT AAA CAC TTC
Gly Met Gly Val Glu Thr Arg Thr Thr Glu Thr Pro Leu Arg Lys His Phe

AAT TTG ACT CCT GTG GGT TCT CAG GGC ATT CGC TTA AGT TGG GAA GTC CAA
Asn Leu Thr Pro Val Gly Ser Gln Gly Ile Arg Leu Ser Trp Glu Val Gln

CAC TTG TCT GAC CTC AAA GGA ACA GAT ATT TCT CTA AAA GCG GTG AAT CCT
His Leu Ser Asp Leu Lys Gly Thr Asp Ile Ser Leu Lys Ala Val Asn Pro

TCT GAC CCG TTA GTC TAC AAA AGA CAA ACT GCA AAA TTC TCA GAT GGA CAA
Ser Asp Pro Leu Val Tyr Lys Arg Gln Thr Ala Lys Phe Ser Asp Gly Gln

CTC ACT ATC GGC GAA CTG AAG CCC TCC ACA TTA TAC AAA ATG ACT GTG GAA
Leu Thr Ile Gly Glu Leu Lys Pro Ser Thr Leu Tyr Lys Met Thr Val Glu

GCA GTG AAA GCG AAA AAG ACC ATT TTG GGA TTC ACC GTA GAC ATT GAG ACA
Ala Val Lys Ala Lys Lys Thr Ile Leu Gly Phe Thr Val Asp Ile Glu Thr

CCG CGC GCT GGC AAG AAG GAA AGC ACT GTA ATG ACT AGT GGA TCC GCC TTA
Pro Arg Ala Gly Lys Lys Glu Ser Thr Val Met Thr Ser Gly Ser Ala Leu

ACA TCC GCA ATC GCT GGT TTT GTA TTC AGC TGC ATA GTG GTT GTC CTT ACT
Thr Ser Ala Ile Ala Gly Phe Val Phe Ser Cys Ile Val Val Val Leu Thr

TGAACTCTCATGTAAGTCAATGCAAATTATCCACTGCTTCTATACTGAGTAGCACGACCCATAACTT

GCATTTTTCAAATAACTCTTCTTCCACATCAGGCTTCCTTGGTGCCGAAGATGCACAAATCACCATT

TATTTTCGCTTTATTAACATTTGTATGACCTCTCATTGTGGATTACTCCCGAATGACAAATACGGGA

CTTTGTCATATTTGCTTCATTGTTATCACCCTTAATTCCAATTCACTGACTCG

14. Molécule d'ADN selon l'une quelconque des revendications 10 à 13 qui est un ADNc.

15. Vecteur d'expression recombinant qui contient une molécule d'ADN selon l'une quelconque des revendications 10 à 14.

16. Cellule hôte transformée par un vecteur selon la revendication 15 capable d'exprimer le polypeptide ou fragment de peptide ou son variant qui est codé.

17. Cellule hôte selon la revendication 16 qui est un procaryote.

18. Cellule hôte selon la revendication 17 où l'hôte procaryote est *E. coli.*

19. Cellule hôte selon la revendication 16, qui est un eucaryote.

20. Méthode de production d'un polypeptide antigénique ou son fragment de peptide ou son variant qui comprend la culture d'une cellule selon l'une quelconque des revendications 16 à 19 et la récupération du produit exprimé.

21. Vaccin contre une infection par *E.granulosus,* comprenant une quantité immunologiquement efficace d'un polypeptide, fragment de peptide ou variant selon l'une quelconque des revendications 1 à 8 en combinaison avec un adjuvant, support ou diluant pharmaceutiquement acceptable pour celui-ci.

22. Vaccin selon la revendication 21, qui contient de plus une quantité efficace d'un ou plusieurs polypeptides de *E.granulosus* ayant un poids moléculaire déterminé par SDS-PAGE sélectionné parmi 30 kD, 34 kD et 40 kD ou bien un ou plusieurs fragments ou variants desdits polypeptides ayant une activité immunologique équivalente à ceux-ci.

23. Vaccin viral recombinant qui contient un acide nucléique codant pour un polypeptide antigénique ou un fragment de peptide ou un variant de celui-ci selon la revendication 1, qui est capable d'exprimer ledit polypeptide codé ou fragment de peptide ou variant.

24. Polypeptide, fragment de peptide ou variant selon l'une quelconque des revendications 1 à 8 pour une utilisation dans une méthode de protection d'un hôte sensible à une infection par un parasite *Echinococcus* ou *Taeniid* contre une telle infection, comprenant l'administration audit hôte, d'une quantité immunologiquement efficace dudit polypeptide, fragment de peptide ou variant.

25. Utilisation d'un polypeptide, fragment de peptide ou variant selon l'une quelconque des revendications 1 à 8 dans la fabrication d'un médicament pour protéger un hôte sensible à une infection par un parasite *Echinococcus* ou *Taeniid* contre une telle infection.

26. Utilisation de la revendication 25, où ledit parasite est *E.granulosus.*

27. Utilisation de la revendication 25, où ledit parasite est *E.multilocularis, E. vogelii, T. ovis, T.* saginata, *T. solium, T; multiceps* ou *T. hydatigena.*

28. Anticorps purifié ou son fragment de liaison spécifique d'un polypeptide antigénique, d'un fragment de peptide ou d'un variant selon la revendication 1.

29. Utilisation d'un anticorps ou son fragment de liaison selon la revendication 28, pour identifier un antigène protecteur d'un parasite *Echinococcus* ou *Taeniid* autre que *E. granulosus.*

30. Molécule d'ADN facultativement marquée comprenant une partie ou la totalité de la séquence de nucléotides de la Figure 4 appropriée pour une utilisation comme sonde pour l'identification de l'acide nucléique codant pour un antigène protecteur d'un parasite *Echinococcus* ou *Taeniid* autre que *E. granulosus.*

31. Utilisation d'une molécule d'ADN selon la revendication 10, pour identifier un ADN codant pour un antigène protecteur d'un parasite *Echinococcus* ou *Taeniid* autre que *E. granulosus.*

32. Utilisation de la revendication 29 ou de la revendication 31, où le parasite autre que *E. granulosus* est *E.multilocularis, E. vogelii, T. ovis. T. saginata, T.* solium, *T. multiceps* ou T. *hydatigena.*

33. Méthode d'identification d'un antigène protecteur d'un parasite *Echinococcus* ou *Taeniid* autre que *E. granulosus* qui comprend l'étape d'identification d'un gène dudit parasite ayant une séquence de nucléotides qui est au moins sensiblement homologue à une partie ou à la totalité de la séquence de nucléotides de la Figure 4.
